# EUROPÄISCHE PATENTSCHRIFT

(11) **EP 3 953 324 B1**
(45) Veröffentlichungstag und Bekanntmachung des Hinweises auf die Patenterteilung: **05.04.2023**
(21) Anmeldenummer: 20709492.1
(22) Anmeldetag: 27.02.2020
(51) Int. Cl.: C07C 381/00, C07D 403/00

(54) **PHOTOREDOXKATALYTISCHE SELEKTIVE AKTIVIERUNG VON SCHWEFELHEXAFLUORID ZUR ALPHA-ALKOXYPENTAFLUORSULFANYLIERUNG VON UNGESÄTTIGTEN KOHLENWASSERSTOFFVERBINDUNGEN SOWIE DERIVATISIERUNG ZU UNGESÄTTIGTEN PENTAFLUORSULFANEN**
PHOTOREDOX CATALYICALLY SELECTIVE ACTIVATION OF SULPHUR HEXAFLUORIDE FOR ALPHA-ALKOXY PENTAFLOUORSULFONATION OF UNSATURATED HYDROCARBON COMPOUNDS AND DERIVATISATION TO FORM UNSATURATED PENTAFLOUORSULFANES
ACTIVATION SÉLECTIVE CATALYTIQUE PHOTOREDOX D'HEXAFLUORURE DE SOUFRE POUR L'ALPHA-ALKOXYPENTAFLUOROSULPHONYLATION DE COMPOSÉS HYDROCARBONÉS INSATURÉS AINSI QUE DÉRIVATISATION EN PENTAFLUOROSULFANES INSATURÉS

(30) Priorität: 09.04.2019 DE 102019109303
(43) Veröffentlichungstag der Anmeldung: 16.02.2022
(73) Patentinhaber: Karlsruher Institut für Technologie, 76131 Karlsruhe (DE)
(72) Erfinder: ROMBACH, David, 8093 Zürich (DE); WAGENKNECHT, Hans-Achim, 75045 Walzbachtal (DE)
(86) Internationale Anmeldenummer: PCT/EP2020/025097
(87) Internationale Veröffentlichungsnummer: WO 2020/207620

(56) Entgegenhaltungen:
- WO-A1-2009/026191
- DAVID ROMBACH ET AL: "Photoredox Catalytic [alpha]-Alkoxypentafluorosulfanylation of [alpha]-Methyl- and [alpha]-Phenylstyrene Using SF 6", ANGEWANDTE CHEMIE, INTERNATIONAL EDITION, Bd. 59, Nr. 1, 2. Januar 2020 (2020-01-02) , Seiten 300-303, XP055696489, DE ISSN: 1433-7851, DOI: 10.1002/anie.201910830
- ROMBACH D ET AL: "Photoredox Catalytic Activation of Sulfur Hexafluoride for Pentafluorosulfanylation of alpha-Methyl and alpha-Phenyl Styrene", CHEMCATCHEM,, Bd. 10, Nr. 14, 4. April 2018 (2018-04-04) , Seiten 2955-2961, XP002786310, in der Anmeldung erwähnt

## Beschreibung

Die vorliegende Erfindung betrifft ein Verfahren zur photoredoxkatalytischen selektiven Aktivierung von Schwefelhexafluorid zur alpha-Alkoxypentafluorsulfanylierung von ungesättigten Kohlenwasserstoffverbindungen sowie ein Verfahren zur Herstellung von ungesättigten Pentafluorsulfanen.

Die organische Synthesechemie stellt sich in zunehmendem Maße der Herausforderung der nachhaltigen Nutzung des Lichtes als Energiequelle für chemische Umsetzungen, beispielsweise des preiswert von LEDs erzeugten Lichtes oder des Sonnenlichtes. In klassischen photochemischen Reaktionen wird die Lichtenergie direkt in das Substrat eingestrahlt oder von einem Sensibilisator absorbiert und übertragen (N. Hoffmann, Chem. Rev. 2008, 108, 1052-1103; A. G. Griesbeck, J. Mattay, a) Photochemical Key Steps in Organic Synthesis, VCH, Weinheim, 1994; b) Synthetic Organic Photochemistry, Marcel Dekker, New York, 2005).

Moderner geprägt ist der Begriff der chemischen Photokatalyse, wobei dessen Gebrauch nicht einheitlich ist. Der Begriff wird immer dann für lichtinduzierte Reaktionen eingesetzt, wenn ein Photokatalysator und nicht das Edukt durch Licht angeregt wird und die Energie oder ein Elektron auf das Edukt (Substrat) oder den Photokatalysator übertragen werden. Grundsätzlich gilt, wie bei jedem Katalysator, dass auch der Photokatalysator nach Entstehung des Produkts unverändert freigesetzt wird und zurückgewonnen werden kann. Ein chemischer Photokatalysator ist ein Stoff (eine Verbindung), der den physikalischen Lichtanregungsprozess mit einer chemischen Folgereaktion zeitlich, räumlich und energetisch koppelt, ggf. unter Nutzung von Zwischenschritten, z. B. bei der Stabilisierung einer Ladungstrennung (M. Fagnoni, D. Dondi, D. Ravelli, A. Albini, Chem. Rev. 2007, 107, 2725-2756).

Die Photokatalyse, insbesondere die Photoredoxkatalyse mit sichtbarem Licht hat in der organischen Synthesechemie in den letzten fünf Jahren stetig zunehmend Anwendungen gefunden. Beispielhaft seien hier einige Übersichtsartikel der Autoren A. Albini (D. Ravelli, D. Dondi, M. Fagnoni, A. Albini, Chem. Soc. Rev. 2009, 38, 1999-2011; D. Ravelli, M. Fagnoni, A. Albini, Chem. Soc. Rev. 2013, 42, 97-113), B. König (D. P. Hari, B. König, Angew. Chem. Int. Ed. 2013, 52, 4734-4743), D. W. C. MacMillan (C. K. Prier, D. A. Rankic, D. W. C. MacMillan, Chem. Rev. 2013, 113, 5322-5363), C. R. J. Stephenson (J. M. R. Naryanam, C. R. J. Stephenson, Chem. Soc. Rev. 2011, 40, 102-113; J. W. Tucker, C. R. J. Stephenson, J. Org. Chem. 2012, 77, 1617-1622), Y. Xi (Y. Xi, H. Yi, A. Lei, Org. Biomol. Chem. 2013, 11, 2387-2403) und T. P. Yoon (T. P. Yoon, M. A. Ischay, J. Du, Nature Chem. 2010, 2, 510-532) erwähnt. Das Repertoire photokatalytischer organischer Reaktionen ist unterdessen extrem breit, beispielhaft seien hier [2+2]-Cycloadditionen, Arylierungen, Cyclisierungen und photoredoxorganokatalytische C-C-Verknüpfungen mit hoher Stereoselektivität genannt.

Üblicherweise umfasst ein Photoredoxkatalysator einen redoxaktiven Chromophor. Bei der Photoredoxkatalyse findet ein Elektronentransfer statt, welcher zur Bildung von Radikalionen führt. Dadurch wird eine organisch-chemische Reaktion initiiert. Der Redoxprozess wird innerhalb des Katalysezyklus durch Rückübertragung des Elektrons (*back electron transfer* (BET)) zum oder vom redoxaktiven Chromophor wieder geschlossen, wodurch der Photoredoxkatalysator am Ende unverändert freigesetzt wird.

Die Darstellung trifluormethylierter Verbindungen durch die Umsetzung von Trichlortoluol mit Antimonpentafluorid durch Swarts im Jahre 1892 (F. Swarts, Acad. Roy. Belg. 1892, 3, 24, 474) legt den Grundstein für die Entwicklung der modernen organischen Fluorchemie. Der pharmakologische Wert fluorierter funktioneller Gruppen wurde am Profil der Trifluormethylgruppe bereits 1927 von F. Lehmann erkannt (F. Lehmann, Pathol. Pharmakol. 1928, 130, 250-255). Während der zurückliegenden 50 Jahre fokussierten sich die Bemühungen im Forschungsfeld der organischen Fluorchemie im Wesentlichen auf die Entwicklung effizienter Methoden zur Darstellung fluorierter sowie trifluormethylierter Verbindungen sowie deren Derivate (beispielsweise Trifluormethylether, Trifluormethylthioether sowie Difluormethylderivate). Während die Chemie der Trifluormethylverbindungen seit den späten 1980er Jahren ein exponentiell ansteigendes Forschungsinteresse auf sich zog, das zur Entwicklung einer Vielzahl von ausgereiften Methoden führte, wurde bis heute nicht über die Entwicklung einer vergleichbar ausgereiften Funktionalisierungsmethode zur Einführung der chalkogenhomologen Pentafluorsulfanylgruppe (R-SF₅ bzw. ·SF₅) berichtet. Die bisher extrem schwierige Zugänglichkeit von Pentafluorsulfanylverbindungen, gepaart mit herausragenden pharmakologischen Eigenschaften, führte dazu, dass die Pentafluorsulfanylgruppe in verschiedenen Arbeiten als "Substituent der Zukunft" betrachtet wird (vgl. X. Mi, J. Chen, L. Xu Eur. J. Org. Chem. 2015, 7, 1415-1418; Scriven, Heterocyclic Chemistry in the 21 st Century: A Tribute to Alan Katritzky, Elsevier 2016, 40; und H. R. A. Golf, H.-U. Reissig, Arno W. J. Org. Chem. 2015, 80, 5133-5143).

In verschiedenen Arbeiten wurde gezeigt, dass die Substitution der Trifluormethylgruppe durch die SFs-Gruppe in vielen Fällen eine Verbesserung des pharmakologischen Wirkprofils erwirkt, was an verschiedenen Eigenschaften des Substituenten nachvollzogen werden kann (vgl. P. R. Savoie, J. T. Welch Chem. Rev. 2015, 115, 1130-1190). Einerseits weist die SFs-Gruppe aufgrund ihrer quadratisch-pyramidalen Geometrie eine verminderte Rotationsbarriere auf und ist aus diesem Grunde in der Lage, mit hoher Bindungskonstante mit biologischen Strukturen zu wechselwirken (vgl. P. R. Savoie, J. T. Welch Chem. Rev. 2015, 115, 1130-1190). Weiterhin vermindert die hohe hydrolytische Beständigkeit der funktionellen Gruppe SF₅ unerwünschte kovalente Enzyminhibition, wie sie im Falle von Trifluormethylverbindungen insbesondere in alkalischem Milieu beobachtet wird. Weiterhin zeichnen sich Verbindungen mit SFs-Gruppe durch einen stärkeren elektronenziehenden Charakter, der sich auf den pKs-Wert umliegender Protonen sowie die Lipophilie der Verbindungen auswirkt, aus. Somit weisen zahlreiche Verbindungen mit SFs-Gruppe eine großes biologisches, medizinisch-chemisches und pharmakologisches Potential auf.

Die derzeit etablierten Methoden zur Einführung des SFs-Substituenten basieren auf der Verwendung hochtoxischer, gasförmiger Reagenzien in frühen Synthesestufen. Hierbei werden die extrem toxischen Verbindungen SF₅Cl und SF₅Br sowie S₂F₁₀ verwendet, die auf dem europäischen Markt teils nicht bzw. nur schlecht verfügbar sind und außerordentlich besonderen Sicherheitsbestimmungen unterliegen.

Diese Restriktionen führen zu einer Unterentwicklung der Methoden zur synthetischen Einführung der SF₅-Gruppe und schließen eine industrielle Nutzung der Methoden kategorisch aus.

In der WO2009026191 wird die Herstellung von gesättigten und ungesättigten vicinalen SF₅-Alkoholen unter Einsatz von SFsBr bzw. SF₅Cl beschrieben.

Die WO2004011422 beschreibt die Addition von SF₅Cl an Phenylacetylen unter Bildung von (E)-1-chloro-2-(pentafluorsulfanyl)-1-phenylethen und (1E,3Z)-1-chloro-1,3-diphenyl-4-pentafluorsulfanylbuta-1,3-dien.

McTeague und Jamison befassen sich mit der photochemischen Aktivierung von Schwefelhexafluorid und der synthetischen Nutzung von SF₆ und beschreiben, wie SF₆ zur Desoxyfluorierung von aktivierten Alkoholen unter stark reduktiven Bedingungen verwendet werden kann, wobei eine Mehrelektronenreduktion des Schwefelhexafluorids stattfindet (T. A. McTeague, T. F. Jamison, Angew. Chem. Int. Ed. 2016, 55, 15072).

Weitere Arbeiten beschäftigen sich mit metallkatalysiertem vollständigen Abbau von Schwefelhexafluorid ohne synthetische Nutzung der Reduktionsprodukte. Zudem wurde die Aktivierung von SF₆ mithilfe von Metallocenen (bspw. Vanadocen) beschrieben (vgl. L. Zámostná, T. Braun, Angew. Chem. Int. Ed. 2015, 54, 10652 - 10656; und B. G. Harvey, A. M. Arif, A. Glöckner, R. D. Ernst, Organometallics 2007, 26 (11), 2872-2879).

In der DE10220901 und der DE10321112 wird ein Verfahren zur Pentafluorsulfanylierung von organischen Verbindungen unter Einsatz von Pentafluorsulfuraniden, die aus SF₆ generiert werden, beschrieben.

Rombach und Wagenknecht beschreiben die photoredoxkatalytische Aktivierung von SF₆ für die Pentafluorsulfanylierung von alpha-Phenylstyrol bzw. alpha-Methylstyrol zur Bildung von vicinalen Pentafluor-(2-fluor-2,2-diphenylethyl)-λ⁶-sulfanen bzw. Pentafluor-(2-fluor-2-phenylpropyl)-λ⁶-sulfanen und die anschließende Eliminierung zu SF₅-Alkenen (vgl. D. Rombach, H.-A. Wagenknecht, ChemCatChem 2018, 10, 2955-2961).

Ein Verfahren zur photochemischen Einführung einer Pentafluorsulfanylgruppe und Bildung von vicinalen Pentafluorsulfanylethern unter Verwendung des ungiftigen Schwefelhexafluorids in organische Substrate (Edukte) und die weitere Derivatisierung von vicinalen Pentafluorsulfanylethern zu ungesättigten Pentafluorsulfanen ist bislang nicht bekannt.

Die Synthese von α-alkoxylierten Sulfanen unter Einsatz von Schwefelhexafluorid ermöglicht eine breite Varietät an Folgereaktionen und erlaubt den Zugang zu neuen Strukturmotiven durch Einführung einer freien Valenz am vicinalen Chalkogensubstituenten an Stelle eines strukturbeendenden vicinalen Halogensubstituenten. Ether stellen zentrale Schlüsselverbindungen in der Synthese der entsprechenden Halogenide oder Ester, Alkene und Alkine, sowie als Vorläufer für sigmatrope Umlagerungreaktionen oder metallorganische C-C-Bindungsknüpfungsreaktionen dar.

Somit liegt der vorliegenden Erfindung die Aufgabe zugrunde, ein Verfahren zur photoredoxkatalytischen selektiven Aktivierung von Schwefelhexafluorid zur alpha-Alkoxypentafluorsulfanylierung eines Edukts mit einer ungesättigten Kohlenstoff-Kohlenstoff-Bindung sowie ein Verfahren zur Herstellung von ungesättigten Pentafluorsulfanen bereitzustellen, welches effizient, nachhaltig und umweltfreundlich sein soll.

Die vorstehende technische Aufgabe wird durch die in den Ansprüchen gekennzeichneten Ausführungsformen gelöst.

In einem ersten Aspekt betriff die vorliegende Erfindung ein Verfahren zur photoredoxkatalytischen selektiven Aktivierung von Schwefelhexafluorid zur alpha-Alkoxypentafluorsulfanylierung eines Edukts mit einer ungesättigten Kohlenstoff-Kohlenstoff-Bindung, umfassend die Schritte
(A) Bereitstellen eines Reaktionsgemisches, welches das Edukt, einen Alkohol ROH, einen Photoredoxkatalysator sowie Schwefelhexafluorid umfasst; und
(B) Bestrahlen des Reaktionsgemisches mit elektromagnetischer Strahlung, welche UV-Licht und/oder Licht aus dem sichtbaren Wellenlängenbereich ist.

Durch das erfindungsgemäße Verfahren werden vicinale Pentafluorsulfanylether ausgehend von ungesättigten Kohlenwasserstoffverbindungen dargestellt. "Darstellung vicinaler Pentafluorsulfanylether" bedeutet erfindungsgemäß, dass an ein Kohlenstoffatom einer ungesättigten Kohlenstoff-Kohlenstoff-Bindung durch das erfindungsgemäße Verfahren eine Pentafluorsulfanylgruppe (SF₅-Gruppe) und an ein anderes Kohlenstoffatom der ungesättigten Kohlenstoff-Kohlenstoff-Bindung eine Alkoxy-Gruppe OR gebunden wird.

Der Rest R ist im erfindungsgemäßen Verfahren eine gesättigte oder ungesättigte, substituierte oder unsubstituierte, unverzweigte oder verzweigte Kohlenwasserstoffgruppe. "Unsubstituiert" bedeutet erfindungsgemäß, dass kein Wasserstoffatom durch einen von einem Wasserstoffatom verschiedenen Substituenten ersetzt ist.

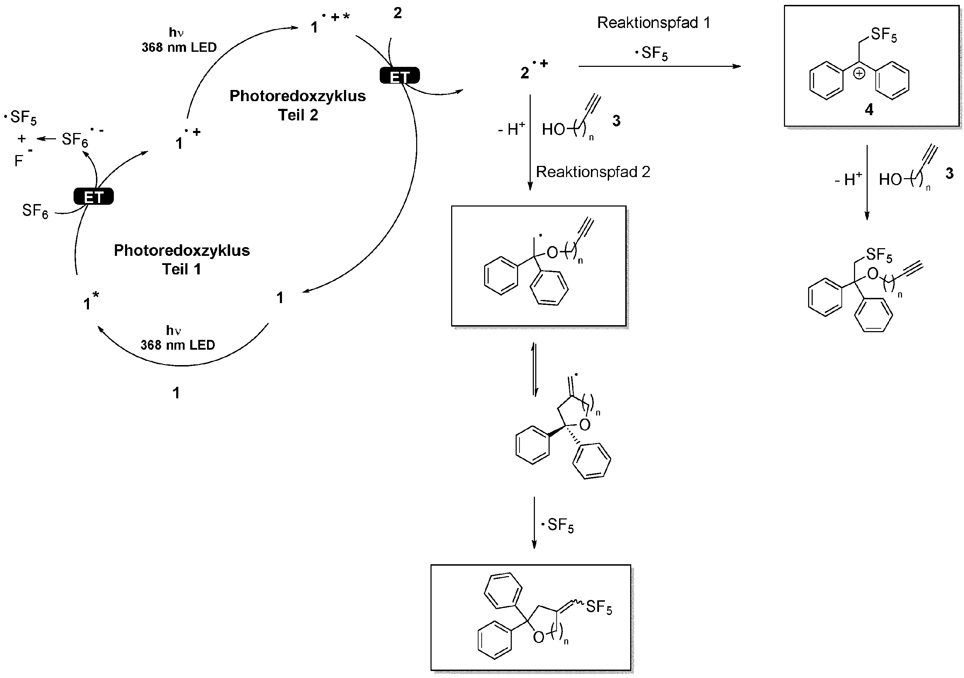

Ohne durch eine bestimmte Theorie eingeschränkt zu sein, wird für das erfindungsgemäße Verfahren folgender Mechanismus angenommen: Durch das Bestrahlen mit elektromagnetischer Strahlung und Anregung durch ein Photon findet ein Einelektronentransfer von dem Photoredoxkatalysator auf SF₆ unter Bildung eines Schwefelhexafluoridradikalanions (·SF₆⁻) (Reduktion) und Oxidation des Photoredoxkatalysators statt. Das Schwefelhhexafluoridradikalanion zerfällt in ein Fluoridanion (F⁻) und ein Pentafluorsulfanylradikal (·SF₅). Das entstandene Radikalkation des Photoredoxkatalysators (Photoredoxkatalysator-Radikalkation) wird durch ein weiteres Photon angeregt und ist nun unter Rückelektronentransfer und Rückbildung des Photoredoxkatalysators in der Lage, das Substrat zum Radikalkation zu oxidieren. Dieses Radikalkation wird durch das Pentafluorsulfanylradikal unter Erzeugung eines (gegebenenfalls stabilisierten) Carbokations abgefangen. Das Carbokation reagiert nun mit dem Alkohol ROH unter Bildung des gewünschten Additionsprodukts (pentafluorsulfanyliertes Produkt) (vgl. Figur 1 und Tabelle 1). Im Kontrast zur Arbeit von McTeague und Jamison (T. A. McTeague, T. F. Jamison, Angew. Chem. Int. Ed. 2016, 55, 15072) basiert die im vorliegenden Verfahren beschriebene Chemie auf einem komplementären Reaktionspfad. In der zitierten Arbeit wird Schwefelhexafluorid zur Deoxyfluorierung von aktivierten Alkoholen wie Allylalkoholen unter Bildung der Allylfluoride eingesetzt. Die Inkompatibilität aktivierter Alkohole mit niederen, aktivierten Schwefelfluorid-Spezies wird in der Arbeit von McTeague und Jamison deutlich. Die Reaktivität von Pentafluorosulfanyl-Anionen basiert auf der Bildung der beiden stabilen Fragmente, des Fluorid-Anions sowie von Schwefeltetrafluorid. Schwefeltetrafluorid ist sehr hydrolyseempfindlich und hydrolysiert zu Schwefeldioxid und Fluorwasserstoff. Interessanterweise erlaubt das vorliegende Verfahren erstmals die Nutzung von sauerstoffhaltigen Reagenzien in der Reaktionsmischung. Das beschriebene Verfahren erlaubt gar die Addition von aktivierten Alkoholen wie die von Allylalkoholen, welche nach aktuellem Stand der Technik in die korrespondierenden Allylfluoride überführt werden. Besonders ist hierbei die komplementäre Reaktivität, die auf der Bildung von Pentafluorsulfanylradikalen an Stelle der korrespondierenden Anionen basiert.

Erfindungsgemäß kann die ungesättigte Kohlenstoff-Kohlenstoff-Bindung sowohl eine Doppel- als auch eine Dreifachbindung sein, wobei eine Doppelbindung bevorzugt ist. Mit dem erfindungsgemäßen Verfahren kann die Alkoxypentafluorsulfanylierung sowohl an einer alleinstehenden Doppel- oder Dreifachbindung erfolgen, als auch an einer Doppelbindung, welche Teil eines konjugierten *π*-Elektronensystems, beispielsweise eines aromatischen Systems, ist. Vorzugsweise erfolgt die Alkoxypentafluorsulfanylierung an einer Kohlenstoff-Kohlenstoff-Doppelbindung, welche Teil eines konjugierten *π*-Elektronensystems, insbesondere eines aromatischen Systems, ist.

Das in dem erfindungsgemäßen Verfahren verwendete Edukt unterliegt keiner besonderen Einschränkung, solange es dem Erfordernis genügt, dass es eine ungesättigte Kohlenstoff-Kohlenstoff-Bindung aufweist. Das Edukt kann beispielsweise eine organische Verbindung sein. Besonders bevorzugt besteht das Edukt aus Atomen, ausgewählt aus der Gruppe, bestehend aus Wasserstoff, Kohlenstoff, Stickstoff, Sauerstoff, Fluor, Brom, Chlor und Schwefel.

Vorzugsweise weist das Edukt ein Reduktionspotential auf, welches kleiner als das Reduktionspotential des Photoredoxkatalysator-Radikalkations ist.

In Bezug auf das Molekülgewicht des Edukts besteht keine besondere Einschränkung. Vorzugsweise beträgt das zahlengemittelte Molekülgewicht des Edukts nicht mehr als 1000 g/mol, besonders bevorzugt nicht mehr als 500 g/mol.

In Schritt (A) des erfindungsgemäßen Verfahrens wird ein Reaktionsgemisch bereitgestellt, welches das Edukt, einen Alkohol ROH, einen Photoredoxkatalysator sowie Schwefelhexafluorid umfasst.

Gemäß der vorliegenden Erfindung unterliegt das Reaktionsgemisch keiner besonderen Einschränkung. Das Reaktionsgemisch kann in einem einheitlichen (in einem einzigen) Aggregatzustand vorliegen. Das heißt, das Reaktionsgemisch kann fest, flüssig oder gasförmig sein, wobei ein flüssiger oder gasförmiger Zustand bevorzugt ist. Es ist allerdings auch möglich, dass das Reaktionsgemisch mehrere Aggregatszustände aufweist. Beispielsweise können das Edukt, der Alkohol ROH und der Photoredoxkatalysator in einem anderen Aggregatszustand vorliegen als das Schwefelhexafluorid.

Gemäß einer bevorzugten Ausführungsform der vorliegenden Erfindung umfasst das Reaktionsgemisch neben dem Edukt, dem Alkohol ROH, dem Photoredoxkatalysator und dem Schwefelhexafluorid zusätzlich ein Lösungsmittel oder ein Lösungsmittelgemisch. Ein geeignetes Lösungsmittel ist beispielsweise Acetonitril. Erfindungsgemäß können deuterierte, teildeuterierte und nicht-deuterierte Lösungsmittel bzw. Lösungsmittelgemische verwendet werden. Das Reaktionsgemisch enthält vorzugsweise 5000 bis 38000 Mol%, besonders bevorzugt 9600 bis 10000 Mol% des Lösungsmittel(gemische)s.

Die relative Stoffmenge des Alkohols ROH im Reaktionsgemisch, bezogen auf die Stoffmenge des Edukts, beträgt vorzugsweise 100 bis 3000 Mol%, besonders bevorzugt 900 bis 1100 Mol%.

Die hierin angegebenen relativen Stoffmengenwerte in "Mol%" beziehen sich auf die Stoffmenge des Edukts im Reaktionsgemisch unmittelbar vor Schritt (B). Konkret entspricht eine absolute Stoffmenge bei einer relativen Stoffmenge von "100 Mol%" der (absoluten) Stoffmenge des Edukts, und bei einer relativen Stoffmenge von "200 Mol%" liegt die zweifache Stoffmenge des Edukts vor.

Gemäß einer besonders bevorzugten Ausführungsform der vorliegenden Erfindung umfasst das Reaktionsgemisch eine Lösung, welche das Edukt, den Photoredoxkatalysator, den Alkohol ROH sowie Schwefelhexafluorid enthält. Insbesondere kann das Reaktionsgemisch aus einer Flüssigphase, gebildet durch eine Lösung, enthaltend das Edukt, den Alkohol ROH, den Photoredoxkatalysator sowie Schwefelhexafluorid, und einer Gasphase, umfassend Schwefelhexafluorid, bestehen. Die Gasphase kann neben Schwefelhexafluorid weitere Bestandteile, beispielsweise ein Inertgas wie Argon oder Stickstoff, enthalten. Vorzugsweise besteht die Gasphase aus Schwefelhexafluorid.

Gemäß der vorliegenden Erfindung ist bevorzugt, dass das Reaktionsgemisch nicht mehr als 50 ppm, vorzugsweise nicht mehr als 5 ppm Sauerstoff enthält. Durch eine niedrige Sauerstoffkonzentration im Reaktionsgemisch kann die Ausbeute an pentafluorsulfanyliertem Produkt erhöht werden.

Gemäß der vorliegenden Erfindung ist bevorzugt, dass das Reaktionsgemisch nicht mehr als 50 ppm, vorzugsweise nicht mehr als 4 ppm Wasser enthält.

Die hierin angegebenen Werte in "ppm" (*parts per million*) beziehen sich auf die Stoffmenge des Lösungsmittels im Reaktionsgemisch unmittelbar vor Schritt (B).

Des Weiteren ist bevorzugt, dass das Reaktionsgemisch weitgehend frei von metallorganischen Verbindungen, wie Metallocenen, beispielsweise Vanadocen oder Ferrocen, ist. Zudem ist bevorzugt, dass das Reaktionsgemisch weitgehend frei von SF₅Cl, SF₅Br und S₂F₁₀ ist, da, wie bereits vorstehend erwähnt, diese Verbindungen toxisch sind. Allerdings kann das erfindungsgemäße Verfahren in Gegenwart der vorstehend aufgeführten Verbindungen durchgeführt werden, da diese unter UV-Bestrahlung photolysieren. Erfindungsgemäß bedeutet "weitgehend frei von einer Verbindung", dass höchstens 50 ppm, vorzugsweise höchstens 5 ppm der Verbindung in dem Reaktionsgemisch enthalten sind.

Gemäß einer bevorzugten Ausführungsform der vorliegenden Erfindung enthält das Reaktionsgemisch weiterhin einen Zusatz, der die Selektivität der Reaktion zum vicinalen Pentafluorsulfanylether erhöht. Erfindungsgemäß ist insbesondere Triethylboran als ein die Selektivität erhöhendes Reagenz bezüglich der Reaktion zum vicinalen Pentafluorsulfanylether geeignet. Die relative Stoffmenge dieses Zusatzes im Reaktionsgemisch, bezogen auf die Stoffmenge des Edukts, beträgt vorzugsweise 0,1 bis 100 Mol%, besonders bevorzugt 10 bis 50 Mol%.

Ohne durch eine bestimmte Theorie beschränkt zu sein, scheint in Schritt (B) Triethylboran die Selektivität der Reaktion dadurch zu erhöhen, dass ein effektives Trapping des *in situ* entstehenden Fluorids durch das Lewis-Säurezentrum von Triethylboran stattfindet, denn ohne den Zusatz von BEt₃ wird eher das vicinale SF₅-F-Produkt gebildet (vgl. I. B. Sivaev, V. I. Bregadze, Coordination Chemistry Reviews 2014, 270-271, 75-88). Andererseits wird Triethylboran in dem erfindungsgemäßen Verfahren durch den Alkohol ROH aktiviert und es entstehen Borsäurespezies, die möglicherweise eine katalytische Funktion übernehmen. Als bekannter Radikalaktivator hat Triethylboran in dem erfindungsgemäßen Verfahren auch einen gewissen Einfluss auf die Radikalkette (vgl. A. Studer, D. P. Curran, Angewandte Chemie 2016, 128, 1, 58-106.)

Somit werden durch den Einsatz von Triethylboran die vicinalen Pentafluorsulfanylether mit deutlich höheren Selektivitäten erhalten.

Der in dem erfindungsgemäßen Verfahren verwendete Photoredoxkatalysator unterliegt keiner besonderen Einschränkung. Geeignete Photoredoxkatalysatoren sind dem Fachmann bekannt.

Die relative Stoffmenge des Photoredoxkatalysators im Reaktionsgemisch beträgt vorzugsweise 0,01 bis 100 Mol%, besonders bevorzugt 1 bis 20 Mol%, bezogen auf die Stoffmenge des Edukts.

Gemäß einer bevorzugten Ausführungsform der vorliegenden Erfindung ist der Photoredoxkatalysator ein Photoredoxkatalysator mit einer N-Phenylphenothiazin-Grundstruktur. Erfindungsgemäß weist der Photoredoxkatalysator dann die Grundstruktur einer bestimmten Verbindung auf, wenn der Photoredoxkatalysator die Verbindung selbst oder ein Derivat davon ist, wobei in dem Derivat ein oder mehrere Wasserstoffatome der Verbindung durch Substituenten ersetzt sind. Diese Substituenten können beispielsweise die gleichen sein, wie nachstehend für die Reste R₁ bis R₁₃ definiert.

Darüber hinaus sind als Photoredoxkatalysator erfindungsgemäß alle organischen und metallorganischen Verbindungen bevorzugt, die eine ähnliche elektronische Struktur wie N-Phenylphenothiazin aufweisen. Eine ähnliche elektronische Struktur liegt beispielsweise dann vor, wenn der Photoredoxkatalysator ein mit dem Reduktionspotential von N-Phenylphenothiazin vergleichbares Reduktionspotential und/oder mit den Absorptionseigenschaften von N-Phenylphenothiazin vergleichbare Absorptionseigenschaften aufweist. Ein vergleichbares Reduktionspotential liegt insbesondere dann vor, wenn der Photoredoxkatalysator das 0,8- bis 1,5-, vorzugsweise das 0,9- bis 1,1-fache Reduktionspotential von N-Phenylphenothiazin aufweist. Vergleichbare Absorptionseigenschaften liegen insbesondere dann vor, wenn die Gesamtabsorption des Photokatalysators im Bereich von 315 bis 500 nm das 0,5- bis 10,0-, vorzugsweise das 0,9- bis 1,1-fache der Gesamtabsorption von N-Phenylphenothiazin im Bereich von 315 bis 500 nm aufweist.

In einer bevorzugten Ausführungsform des erfindungsgemäßen Verfahrens umfasst oder ist der Photoredoxkatalysator eine Verbindung der folgenden Formel (1) 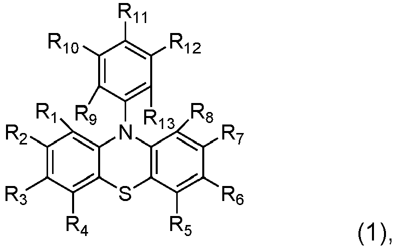 wobei die Reste R₁ bis R₁₃ unabhängig voneinander ein Wasserstoffatom, ein Deuteriumatom, ein Halogenatom, eine Cyanogruppe, eine Nitrogruppe, eine Trifluormethylgruppe, ein Fluoratom, eine substituierte oder unsubstituierte Kohlenwasserstoffgruppe, welche gegebenenfalls ein oder mehrere Heteroatome enthält, oder einen substituierten oder unsubstituierten aromatischen Rest darstellen.

Die Reste R₁ bis R₁₃ sind unabhängig voneinander vorzugsweise ein Wasserstoffatom, ein Deuteriumatom, eine substituierte oder unsubstituierte Kohlenwasserstoffgruppe, welche gegebenenfalls ein oder mehrere Heteroatome enthält, oder ein substituierter oder unsubstituierter aromatischer Rest.

Erfindungsgemäß ist besonders bevorzugt, dass der Photoredoxkatalysator N-Phenylphenothiazin umfasst bzw. N-Phenylphenothiazin ist. In diesem Fall ist jedes von R₁ bis R₁₃ der vorstehenden Formel (1) ein Wasserstoffatom.

Gemäß einer bevorzugten Ausführungsform der vorliegenden Erfindung umfasst bzw. ist das Edukt eine Verbindung der folgenden Formel (I) 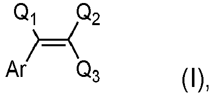 wobei die Reste Q₁ bis Q₃ unabhängig voneinander ein Wasserstoffatom, ein Deuteriumatom, eine Cyanogruppe, eine Trifluormethylgruppe, eine substituierte oder unsubstituierte Kohlenwasserstoffgruppe, welche gegebenenfalls ein oder mehrere Heteroatome enthält, oder einen substituierten oder unsubstituierten aromatischen Rest darstellen und Ar ein aromatischer Rest ist.

Vorzugsweise ist der aromatische Rest Ar eine substituierte oder unsubstituierte aromatische Kohlenwasserstoffgruppe, eine substituierte oder unsubstituierte aromatische heterocyclische Gruppe, eine substituierte oder unsubstituierte kondensierte polycyclische aromatische Gruppe, wobei eine substituierte oder unsubstituierte aromatische Kohlenwasserstoffgruppe bevorzugt ist. Gemäß einer besonders bevorzugten Ausführungsform sind der aromatische Rest Ar und der Rest Q₁ unabhängig voneinander jeweils eine substituierte oder unsubstituierte Phenylgruppe, wobei am meisten bevorzugt ist, dass jedes von Ar und Q₁ eine unsubstituierte Phenylgruppe ist.

"Unsubstituiert" bedeutet erfindungsgemäß, dass kein Wasserstoffatom durch einen von einem Wasserstoffatom verschiedenen Substituenten ersetzt ist.

Die substituierte oder unsubstituierte, gegebenenfalls ein oder mehrere Heteroatome enthaltende Kohlenwasserstoffgruppe der vorstehenden Formel (1) bzw. der vorherstehenden Formel (I) kann beispielsweise ein geradkettiges oder verzweigtes Alkyl mit 1 bis 12 Kohlenstoffatomen, welche einen Substituenten aufweisen können, Cycloalkyl mit 3 bis 12 Kohlenstoffatomen, welche einen Substituenten aufweisen können, geradkettiges oder verzweigtes Alkenyl mit 1 bis 6 Kohlenstoffatomen, welche einen Substituenten aufweisen können, geradkettiges oder verzweigtes Alkyloxy mit 1 bis 12 Kohlenstoffatomen, welche einen Substituenten aufweisen können, oder Cycloalkyloxy mit 5 bis 10 Kohlenstoffatomen, welche einen Substituenten aufweisen können, sein. Sofern ein Heteroatom vorhanden ist, ist dieses vorzugsweise aus der Gruppe, bestehend aus Sauerstoff, Stickstoff und Schwefel, ausgewählt.

Gemäß einer bevorzugten Ausführungsform der vorliegenden Erfindung ist das Edukt eine Verbindung mit einer Styrol-Grundstruktur. Für Edukte mit Styrol-Grundstruktur wird durch das erfindungsgemäße Verfahren das pentafluorsulfanylierte Produkt mit besonders hoher Ausbeute erhalten.

Gemäß einer bevorzugten Ausführungsform der vorliegenden Erfindung umfasst bzw. ist das Edukt eine Verbindung der folgenden Formel (II) 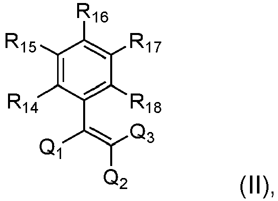 wobei für die Reste Q₁, Q₂ und Q₃ von Formel (II) das gleiche wie für die Reste Q₁, Q₂ und Q₃ von Formel (I) gilt und für die Reste R₁₄ bis R₁₈ das gleiche wie für die Reste R₁ bis R₁₃ von Formel (1) gilt. Vorzugsweise ist jedes von R₁₄ bis R₁₈ ein Wasserstoffatom. Zudem ist bevorzugt, dass Q₁ eine unsubstituierte Phenylgruppe ist.

In Schritt (B) des erfindungsgemäßen Verfahrens wird das Reaktionsgemisch mit elektromagnetischer Strahlung bestrahlt. Erfindungsgemäß ist die elektromagnetische Strahlung UV-Licht (d. h. elektromagnetische Strahlung mit einer Wellenlänge von 100 nm bis kleiner als 400 nm) und/oder Licht aus dem sichtbaren Wellenlängenbereich (d. h. elektromagnetische Strahlung mit einer Wellenlänge von 400 bis 800 nm).

Die in Schritt (B) verwendete elektromagnetische Strahlung kann sowohl monochromatisches Licht als auch Licht (elektromagnetische Strahlung), welches eine Überlagerung von elektromagnetischer Strahlung verschiedener Wellenlängen darstellt, sein. Vorzugsweise wird monochromatisches Licht oder schmalbandiges Licht, beispielsweise LED-Licht, verwendet. "Schmalbandig" bedeutet in diesem Zusammenhang, dass die elektromagnetische Strahlung einen Wellenlängenbereich von höchstens 200 nm, bevorzugt höchstens 150 nm, insbesondere bevorzugt höchstens 50 nm abdeckt. Gemäß einer bevorzugten Ausführungsform des erfindungsgemäßen Verfahrens ist die elektromagnetische Strahlung UV-A Licht und/oder Licht aus dem sichtbaren Wellenlängenbereich mit einer Wellenlänge von 400 bis 600 nm. Erfindungsgemäß weist UV-A-Licht eine Wellenlänge von mindestens 315 nm bis weniger als 400 nm auf. Das heißt, es ist bevorzugt, dass die Wellenlänge der elektromagnetischen Strahlung 315 bis 500 nm beträgt. Insbesondere ist bevorzugt, dass die Wellenlänge der in Schritt (B) verwendeten elektromagnetischen Strahlung 350 bis 400 nm beträgt. Bei Verwendung von elektromagnetischer Strahlung aus vorstehenden Wellenlängenbereichen werden mit dem erfindungsgemäßen Verfahren besonders hohe Ausbeuten an pentafluorsulfanyliertem Produkt erhalten.

Bevorzugte Lichtleistungen liegen zwischen 500 und 2000 mW. Bevorzugte Lichtquellen sind High-Power-LEDs. Weiterhin können konventionelle Energiesparlampen sowie Hg-Lampen, gegebenenfalls unter Verwendung von Filtern, verwendet werden.

In einem weiteren Aspekt betrifft die vorliegende Erfindung ein Verfahren zur photoredoxkatalytischen selektiven Aktivierung von Schwefelhexafluorid zur Darstellung von ((Pentafluorsulfanyl)-methylen)-oxacycloalkanen unter Verwendung eines Edukts mit einer ungesättigten Kohlenstoff-Kohlenstoff-Bindung sowie eines Alkohols ROH mit einer terminalen Alkingruppe R = HCC(CH₂)ₙ- mit n > 1, umfassend die Schritte
(Aa) Bereitstellen eines Reaktionsgemisches, welches das Edukt, einen Alkohol ROH mit einer terminalen Alkingruppe R = HCC(CH₂)ₙ- mit n > 1, einen Photoredoxkatalysator sowie Schwefelhexafluorid umfasst; und
(Bb) Bestrahlen des Reaktionsgemisches mit elektromagnetischer Strahlung, welche UV-Licht und/oder Licht aus dem sichtbaren Wellenlängenbereich ist.

((Pentafluorsulfanyl)-methylen)-oxacycloalkane bedeutet erfindungsgemäß, dass an ein Kohlenstoffatom der ungesättigten Kohlenstoff-Kohlenstoff-Bindung eine Alkoxy-Gruppe OR mit R = HCC(CH₂)ₙ- und n > 1 gebunden wird, an das andere Kohlenstoffatom die endständige Alkingruppe addiert wird und durch das erfindungsgemäße Verfahren eine Pentafluorsulfanylgruppe (SF₅-Gruppe) an die entstehende exocyclische Methylengruppierung addiert wird.

Ohne durch eine bestimmte Theorie eingeschränkt zu sein, wird für das erfindungsgemäße Verfahren folgender Mechanismus angenommen: Durch das Bestrahlen mit elektromagnetischer Strahlung und Anregung durch ein Photon findet ein Einelektronentransfer von dem Photoredoxkatalysator auf SF₆ unter Bildung eines Schwefelhexafluoridradikalanions (·SF₆-) (Reduktion) und Oxidation des Photoredoxkatalysators statt. Das Schwefelhexafluoridradikalanion zerfällt in ein Fluoridanion (F-) und ein Pentafluorsulfanylradikal (·SF₅). Das entstandene Radikalkation des Photoredoxkatalysators (Photredoxkatalysator-Radikalkation) wird durch ein weiteres Photon angeregt und ist nun unter Rückelektronentransfer und Rückbildung des Photoredoxkatalysators in der Lage, das Substrat zum Radikalkation zu oxidieren. Dieses Radikalkation wird durch die Hydroxyfunktion des Alkinols angegriffen und erzeugt ein terminales Methylenradikal, welches im Gleichgewicht mit einem geschlossenen Vinylradikal liegt. An das geschlossene Vinylradikal addiert im letzten Schritt das zuvor gebildete Pentafluorsulfanylradikal unter Bildung eines ((Pentafluorsulfanyl)-methylen)-oxacycloalkans. In dieser Reaktion konkurriert die Addition des Alkinols unter Bildung eines terminalen Methylenradikals mit der Radikal-Radikal-Rekombination zwischen Ethylenradikalkation und dem gebildeten SF₅-Radikal (vgl. Figur 1 und Tabelle 1).

Figur 1 beschreibt schematisch den Reaktionsmechanismus der konkurrierenden Bildung der ((Pentafluorsulfanyl)-methylen)-oxacycloalkane und Pentafluor-(2-alkinoxyethyl)-sulfane unter Verwendung der Beispielstrukturen **1** und **2** (siehe Tabelle 1) sowie eines Alkinols **3**. Der in Figur 1 dargestellte, zugrundeliegende Photoredoxzyklus beginnt mit einem Einelektronentransfer (ET) vom Photoredoxkatalysator **1** auf SF₆ unter Bildung eines Schwefelhexafluoridradikalanions (·SF₆⁻) (Reduktion), eines Fluoridions und eines Photoredoxkatalysator-Radikalkations **1^{·+}** (Oxidation) durch das Bestrahlen mit elektromagnetischer Strahlung und Anregung durch ein Photon (Photoredoxzyklus Teil 1). Die anschließende Lichtanregung des erzeugten Photoredoxkatalysator-Radikalkations **1^{·+}** oxidiert das Substrat **2** unter Bildung des zentralen Radikalkations **2^{·+}** und Rückbildung des Photoredoxkatalysators **1** (Photoredoxzyklus Teil 2). Nach Reaktionspfad 1 wird das Radikalkation **2^{·+}** durch das Pentafluorsulfanylradikal unter Erzeugung eines SFs-Carbokations **4** abgefangen; das Carbokation **4** reagiert nun mit dem Alkinol **3** unter Bildung des vicinalen SF5-Ethers. Nach Reaktionspfad 2 wird das Radikalkation **2^{·+}** durch die Hydroxyfunktion des Alkinols **3** angegriffen und erzeugt ein terminales Methylenradikal, welches im Gleichgewicht mit einem geschlossenen Vinylradikal liegt. An das geschlossene Vinylradikal addiert im letzten Schritt das zuvor gebildete Pentafluorsulfanylradikal unter Bildung eines ((Pentafluorsulfanyl)-methylen)-oxacycloalkans.

**Tabelle 1**

| Beispielstruktur Nr. | Chemische Formel |
|---|---|
| 1 | 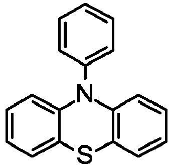 |
| 2 | 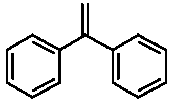 |
| 3 | 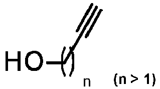 |
| 4 | 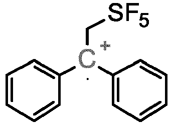 |

Ein weiterer Aspekt der vorliegenden Erfindung betrifft ein Verfahren zur Eliminierung von gemäß den Schritten (A) und (B) hergestellten Pentafluor-(2-alkoxyethyl)-λ⁶-sulfanen zu Pentafluor-(ethylen)-λ⁶-sulfanen, umfassend die folgenden Schritte:
(C) Bereitstellen eines Reaktionsgemisches, welches als Edukt einen vicinalen Pentafluorsulfanylether, eine starke Lewis-Säure und ein Lösungsmittel umfasst; und
(D) Rühren des Reaktionsgemisches; und
(E) Quenchen des Reaktionsgemisches mit einer wässrigen Lösung und Extraktion des Produktes mit einem organischen Lösungsmittel.

Durch das erfindungsgemäße Verfahren werden Pentafluorsulfanylalkene ausgehend von vicinalen Pentafluorsulfanylethern unter Zusatz einer starken Lewis-Säure dargestellt. Gemäß einer bevorzugten Ausführungsform der vorliegenden Erfindung wird als starke Lewis-Säure BF₃·Et₂O gewählt (vgl. G. H. Posner, E. M. Shulman-Roskes, C. Ho Oh, J.-C. Carry, J. V. Green, A. B. Clark, H. Dai, T. E. N. Anjeh, Tetrahedron Letters 1991, 32, 6489-6492). Die Pentafluorsulfanyl-Gruppe wird hierbei von BF₃·Et₂O nicht angegriffen und bleibt stabil. Die Besonderheit der Darstellung der Alkene aus Ethern besteht in der Lockerung der strikten Reaktionsbedingungen zur Darstellung der Pentafluorsulfanyl-substituierten Fluoride in Bezug auf Wasserfreiheit, was die industrielle Nutzung stark vereinfacht und die Prozesskosten reduziert. Die gewünschten Moleküle werden nun auch über die Ether erhalten oder können gar aus einem Reaktionsgemisch von Fluoriden und Ethern unter identischen Reaktionsbedingungen erhalten werden, was die Ausbeute der Produkte erhöht und beide Reaktionskanäle in denselben Produktkanal einmünden lässt.

In Bezug auf das Molekülgewicht des Edukts besteht keine besondere Einschränkung. Vorzugsweise beträgt das zahlengemittelte Molekülgewicht des Edukts nicht mehr als 1000 g/mol, besonders bevorzugt nicht mehr als 500 g/mol.

Gemäß einer bevorzugten Ausführungsform der vorliegenden Erfindung ist das Lösungsmittel ein reines Lösungsmittel oder ein Lösungsmittelgemisch. Ein geeignetes Lösungsmittel ist beispielsweise Chloroform. Erfindungsgemäß können deuterierte, teildeuterierte und nicht-deuterierte Lösungsmittel bzw. Lösungsmittelgemische verwendet werden. Das Reaktionsgemisch enthält vorzugsweise 1 000 bis 1 000 000 Mol%, besonders bevorzugt 50 000 bis 100 000 Mol% des Lösungsmittel(gemische)s.

Die relative Stoffmenge des BF₃·Et₂O im Reaktionsgemisch beträgt vorzugsweise 500 - 5000 Mol%, besonders bevorzugt 3300 Mol%, bezogen auf die Stoffmenge des Edukts.

Gemäß einer bevorzugten Ausführungsform der vorliegenden Erfindung umfasst bzw. ist das Edukt eine Verbindung der folgenden Formel (III) 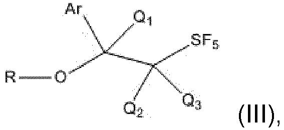 wobei Ar ein aromatischer Rest ist, die Reste Q₁ bis Q₃ unabhängig voneinander ein Wasserstoffatom, ein Deuteriumatom, eine Cyanogruppe, eine Trifluormethylgruppe, eine substituierte oder unsubstituierte Kohlenwasserstoffgruppe, welche gegebenenfalls ein oder mehrere Heteroatome enthält, oder einen substituierten oder unsubstituierten aromatischen Rest darstellen. R ist ausgewählt aus einer gesättigten oder ungesättigten, substituierten oder unsubstituierten, unverzweigten oder verzweigten Kohlenwasserstoffgruppe.

Vorzugsweise ist der aromatische Rest Ar eine substituierte oder unsubstituierte aromatische Kohlenwasserstoffgruppe, eine substituierte oder unsubstituierte aromatische heterocyclische Gruppe, eine substituierte oder unsubstituierte kondensierte polycyclische aromatische Gruppe, wobei eine substituierte oder unsubstituierte aromatische Kohlenwasserstoffgruppe bevorzugt ist. Gemäß einer besonders bevorzugten Ausführungsform sind der aromatische Rest Ar und der Rest Q₁ unabhängig voneinander jeweils eine substituierte oder unsubstituierte Phenylgruppe, wobei am meisten bevorzugt ist, dass jedes von Ar und Q₁ eine unsubstituierte Phenylgruppe ist.

"Unsubstituiert" bedeutet erfindungsgemäß, dass kein Wasserstoffatom durch einen von einem Wasserstoffatom verschiedenen Substituenten ersetzt ist.

Die substituierte oder unsubstituierte, gegebenenfalls ein oder mehrere Heteroatome enthaltende Kohlenwasserstoffgruppe der vorstehenden Formel (III) kann beispielsweise ein geradkettiges oder verzweigtes Alkyl mit 1 bis 6 Kohlenstoffatomen, welche einen Substituenten aufweisen können, Cycloalkyl mit 5 bis 10 Kohlenstoffatomen, welche einen Substituenten aufweisen können, geradkettiges oder verzweigtes Alkenyl mit 2 bis 6 Kohlenstoffatomen, welche einen Substituenten aufweisen können, geradkettiges oder verzweigtes Alkyloxy mit 1 bis 6 Kohlenstoffatomen, welche einen Substituenten aufweisen können, oder Cycloalkyloxy mit 5 bis 10 Kohlenstoffatomen, welche einen Substituenten aufweisen können, sein. Sofern ein Heteroatom vorhanden ist, ist dieses vorzugsweise aus der Gruppe, bestehend aus Sauerstoff, Stickstoff und Schwefel, ausgewählt.

Gemäß einer bevorzugten Ausführungsform der vorliegenden Erfindung ist das Edukt eine Verbindung mit einer Styrol-Grundstruktur. Für Edukte mit Styrol-Grundstruktur wird durch das erfindungsgemäße Verfahren das pentafluorsulfanylierte Produkt mit besonders hoher Ausbeute erhalten.

Gemäß einer bevorzugten Ausführungsform der vorliegenden Erfindung umfasst bzw. ist das Edukt eine Verbindung der folgenden Formel (IV) 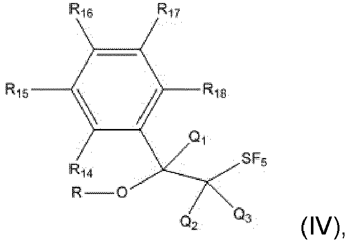 wobei für die Reste Q₁, Q₂, Q₃ und R von Formel (IV) das gleiche wie für die Reste Q₁, Q₂, Q₃ und R von Formel (III) gilt und für die Reste R₁₄ bis R₁₈ das gleiche wie für die Reste R₁ bis R₁₃ von Formel (1) gilt. Vorzugsweise ist jedes von R₁₄ bis R₁₈ ein Wasserstoffatom. Zudem ist bevorzugt, dass Q₁ eine unsubstituierte Phenylgruppe ist.

Die vorliegende Erfindung wird anhand der folgenden, nicht einschränkenden Beispiele näher erläutert.

### Beispiele

**Alpha-Alkoxypentafluorsulfanylierung von** 1,1-Diphenylethylen bzw. von α-Methylstyrol

### Beispiel 1: Pentafluor-(2-methoxy-2,2-diphenylethyl)-λ⁶-sulfan

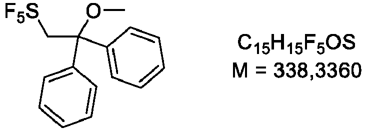

In einem Glasrohr mit Young-Hahn wurden 35,3 µL (36,1 mg, 0,20 mmol, 1,00 eq.) 1,1-Diphenylethylen sowie 81,1 µL (64,1 mg, 2,00 mmol, 10,0 eq.) Methanol in 1,0 mL Sureseal^{®} Acetonitril^{®} gelöst. Anschließend wurden 5,5 mg (0,2 mmol, 10 mol%) N-Phenylphenothiazin sowie 20 µL Triethylboran zugegeben. Die Reaktion wurde durch drei *freeze-pump-thaw-Zyklen* entgast und die Atmosphäre wurde gegen Schwefelhexafluorid getauscht. Die Reaktionsmischung wurde für 22 h bei 20°C unter Belichtung bei 368 nm gerührt. Das Reaktionsprodukt wurde durch NMR-Spektroskopie quantifiziert und durch Säulenchromatographie aufgereinigt (Silica, 10% DCM in Hexan). Das Produkt wurde als farbloser Feststoff (50%) erhalten.

**¹H NMR** (500 MHz, Chloroform-*d*) δ 7.33 - 7.28 (m, 8H, Ar**H**), 7.26 - 7.22 (m, 2H, ArH), 4.72 (p, *J* = 7.9 Hz, 2H, **CH₂**-SF₅), 3.18 (s, 3H, **CH₃**).

**¹³C NMR** (126 MHz, Chloroform-*d*) δ 142.90 (**C**_{q,Ar}), 128.37 (**C**H_{Ar}), 127.52(**C**H_{Ar}), 126.60 (**C**H_{A}), 81.34 (**C**_{q,Brücke}), 74.80 (p, *J* = 10.7 Hz, **C**H₂SF₅), 51.36 (O**C**H₃).

**¹⁹F NMR** (471 MHz, Chloroform-*d*) δ 84.36 (p, *J* = 147.8 Hz, 1 **Fₐₓ**), 71.90 (dt, *J* = 148.9, 8.1 Hz, 4 **F_{aeq}**).

**HR-EI-MS** m/z (ber.) = 338.0764 [M^{·+}]; m/z (gef.) = 338.0765 [M^{·+}] (C₁₅H₁₅F₅OS).

### Beispiel 2: Pentafluor-(2-isopropoxy-2-phenylpropyl)-λ⁶-sulfan

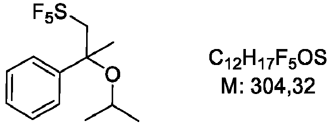

In einem Glasrohr mit Young-Hahn wurden 23,1 µl (21,0 mg, 0,178 mmol, 1,00 eq.) α-Methylstyrol sowie 154 µl (2,00 mmol, 11,2 eq.) Isopropanol in 1 ml filtriertem Sureseal^{®} Acetonitril gelöst. Anschließend wurden 5,5 mg (0,02 mmol, 11 mol%) N-Phenylphenothiazin zugegeben und 20 µl (0,20 mmol, 11 mol%) Triethylboran (1M in Hexan) zur Reaktionsmischung gegeben. Die Reaktionsmischung wurde unter Anwendung von drei *freeze-pump-thaw-Zyklen* entgast. Anschließend wurde die Atmoshphäre gegen Schwefelhexafluorid getauscht und die Reaktionsmischung für 22 h bei 20°C unter Belichtung bei 368 nm gerührt. Das Reaktionsprodukt wurde durch ¹⁹F-NMR-Spektroskopie quantifiziert Das Produkt wurde durch Säulenchromatographie (Silica, Hexan, Rf (Hexan) = 0.4) gereinigt und als farbloses viskoses Öl erhalten (38%).

**¹H** NMR (500 MHz, Chloroform-*d*) δ 7.50 (d, *J* = 7.7 Hz, 2H, C**H**_{Ar}), 7.36 (t, *J* = 7.4 Hz, 2H, C**H**_{Ar}), 7.31 (t, *J* = 7.3 Hz, 1H, C**H**_{Ar}), 4.00 - 3.89 (m, 1H, **CH₂**SF₅), 3.89 - 3.75 (m, 1H, **CH₂**SF₅), 3.55 (h, *J* = 6.1 Hz, 1H, C**H**iPr), 1.91 (s, 3H, **CH₃**), 1.15 (d, *J* = 6.1 Hz, 3H, CH**CH₃**), 0.93 (d, *J* = 6.1 Hz,3H, CH**CH₃**).

**¹³C NMR** (126 MHz, Chloroform-d) δ 143.06 (**C**_{q,Ar}), 128.32 (**CH**_{Ar}), 128.19 (**CH**_{Ar}, 127.05 (**CH**_{Ar}), 77.88 (**C**_{**q**,Brücke}) , 66.09 (**CH₂**-SF5) , 24.88 (**CH₃**-iPr) , 24.36 (**CH₃**-iPr) , 22.00 (**CH₃**).

**¹⁹F NMR** (471 MHz, Chloroform-*d*) δ 86.78 - 84.64 (m, 1H, 1 **Fₐₓ**), 70.05 (dt, *J* = 146.5, 8.9 Hz, 4 **F_{aeq}**).

**HR-EI-MS** m/z (calc.) = 304.0920 [M'+]; m/z (ber.) = 303.0842 [M-H^{·+}]; m/z (found) = 303.0842 [M-H^{·+}], m/z (ber.) = 245.0423 [M-OiPr^{·+}]; m/z (gef.) = 245.0424 [M-OiPr^{·+}].

### Beispiel 3: Pentafluor-(2-(pent-3-in-1-yloxy)-2,2-diphenylethyl)-λ⁶-sulfan

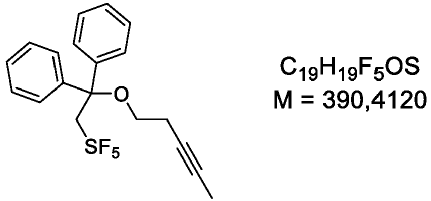

In einem Glasrohr mit Young-Hahn wurden 35,3 µl (36,1 mg, 0,20 mmol, 1,00 eq.) 1,1-Diphenylethylen sowie 93.4 µl (1,00 mmol, 10,0 eq.) 3-Pentin-1-ol in 1 ml filtriertem Sureseal^{®} Acetonitril gelöst. Anschließend wurden 1,4 mg (0,05 mmol, 5 mol%) N-Phenylphenothiazin zugegeben und 10 µl Triethylboran. Die Atmosphäre wurde gegen Schwefelhexafluorid getauscht und die Reaktionsmischung wurde für 21 h bei 20°C unter Belichtung bei 368 nm gerührt. Das Reaktionsprodukt wurde durch NMR-Spektroskopie quantifiziert und durch Säulenchromatographie aufgereinigt (Silica, 10% DCM in Hexan). Das Produkt wurde als hochviskoses Öl (42%) erhalten.

**¹H NMR** (500 MHz, Chloroform-*d*) δ 7.33 - 7.27 (m, 8H, C**H**_{Ar}), 7.23 (m, 2H, C**H**_{Ar}), 4.72 (p, *J* = 7.8 Hz, 2H, **CH₂**-SF₅), 3.31 (t, *J* = 7.4 Hz, 2H, **O-CH₂)**, 2.48 (tq, *J* = 7.4, 2.6 Hz, 2H, **CH₂**-C≡C), 1.75 (t, *J* = 2.5 Hz, 3H, C≡C-**CH₃**).

**¹³C NMR (**126 MHz, Chloroform-*d*) δ 143.04 (**C**_{q,Ar}) , 128.36 (**CH**_{Ar}), 127.56 (**CH**_{Ar}), 126.57 (**CH**_{Ar}), 80.76 (**C**_{**q**,Brücke}) , 76.96 (**C**_{C≡C}), 75.64 (**C**_{C≡C}) , 75.21 (p, *J* = 11.4 Hz, **CH₂**-SF₅), 62.14 (O-**CH₂**) , 20.22 (-**CH₂**-C≡C), 3.57 (C≡C-**CH₃**) .

**¹⁹F NMR** (471 MHz, Chloroform-*d*) δ 84.14 (p, *J* = 147.7, 1 **Fₐₓ**), 72.06 (dt, *J* = 148.8, 8.0 Hz, 4 **F_{aeq}**).

**HR-EI-MS** m/z (calc.) = 390.1077 [M'+]; m/z (found) = 390.1075 [M^{·+}] (C₁₉H₁₉F₅OS). Beispiel 4: **Pentafluor-(2-(pent-4-in-1-yloxy)-2,2-diphenylethyl)-λ⁶-sulfan** 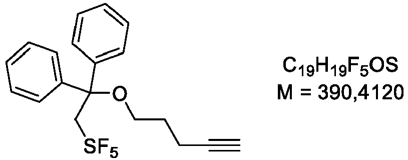 In einem Glasrohr mit Young-Hahn wurden 35,3 µl (36,1 mg, 0,20 mmol, 1,00 eq.) 1,1-Diphenylethylen sowie 186 µl (168 mg, 2,00 mmol, 10,0 eq.) 4-Pentin-1-ol in 1 ml Sureseal^{®} Acetonitril (filtriert) gelöst. Anschließend wurden 5,5 mg (0,2 mmol, 10 mol%) N-Phenylphenothiazin zugegeben sowie 20 µl Triethylboran. Die Atmosphäre wurde gegen Schwefelhexafluorid getauscht und die Reaktionsmischung wurde für 22 h bei 20°C unter Belichtung bei 368 nm gerührt. Das Reaktionsprodukt wurde durch NMR-Spektroskopie quantifiziert und durch Säulenchromatographie aufgereinigt (Silica, 10% DCM in Hexan). Das Produkt wurde als hochviskoses Öl (17%) erhalten.

**¹H NMR** (500 MHz, Chloroform-*d*) δ 7.32-7.13 (m, 10H, C**H**_{Ar}) 4.74 (p, *J* = 7.8 Hz, 1H, **CH₂**-SF₅), 3.31 (t, *J* = 5.9 Hz, 1H, **O-CH₂)**, 2.39 (td, *J* = 7.3, 2.7 Hz, 2H,**CH₂**-C≡C), 1.91 (t, *J* = 2.7 Hz, 1H, C≡C-**H**), 1.89 - 1.83 (m, 2H, CH₂**CH₂**CH₂).

**¹³C NMR** (126 MHz, Chloroform-*d*) δ 143.04 (**C**_{q,Ar}) , 128.36 (**CH**_{Ar}), 127.56 (**CH**_{Ar}), 126.57 (**CH**_{Ar}), 80.76 (**C**_{q,Brücke}) , 76.96 (C_{C≡C}), 75.64 (**C**_{C≡C}) , 75.21 (p, *J* = 11.4 Hz, **CH₂**-SF₅), 62.14 (O-**CH₂**) , 20.22 (**-CH₂**-C≡C), 3.57 (C≡C-**CH₃**).

**¹⁹F NMR** (471 MHz, Chloroform-*d*) δ 84.43 (p, *J* = 147.2 Hz, **Fₐₓ**), 72.10 (dt, *J* = 148.9, 8.0 Hz, 4 **F_{aeq}**).

**HR-EI-MS** m/z (calc.) = 390.1077 [M'+]; m/z (found) = 390.1077 [M˙⁺] (C₁₉H₁₉F₅OS).

### Beispiel 5: Pentafluor-(2-(but-3-in-1-yloxy)-2,2-diphenylethyl)-λ⁶-sulfan

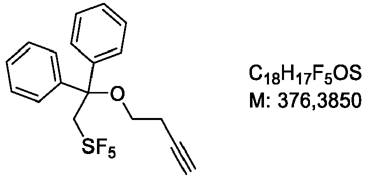

In einem Glasrohr mit Young-Hahn wurden 35,3 µl (36,1 mg, 0,20 mmol, 1,00 eq.) 1,1-Diphenylethylen sowie 151 µl (140 mg, 2,00 mmol, 10,0 eq.) 3-Butin-1-ol in 1 ml Sureseal^{®} Acetonitril (filtriert) gelöst. Anschließend wurden 5,5 mg (0,2 mmol, 10 mol%) N-Phenylphenothiazin zugegeben sowie 20 µl Triethylboran. Die Atmosphäre wurde gegen Schwefelhexafluorid getauscht und die Reaktionsmischung wurde für 22 h bei 20°C unter Belichtung bei 368 nm gerührt. Das Reaktionsprodukt wurde durch NMR-Spektroskopie quantifiziert und durch Säulenchromatographie aufgereinigt (Silica, 5% DCM in Hexan). Das Produkt wurde als hochviskoses Öl (17%) erhalten.

**¹H NMR** (500 MHz, CDCl3) δ = 7.35 - 7.21 (m, 10H, C**H**_{Ar}), 4.73 (p, J=7.8, 2H, **CH₂**-SFs), 3.36 (t, J=7.1, 2H, **O-CH₂)**, 2.54 (td, J=7.1, 2.7, 2H, **CH₂**-C≡C), 1.97 (t, J=2.7, 1H, C≡C-**H**).

**¹³C NMR** (126 MHz, CDCl3) δ = 142.88 (**C**_{q,Ar}), 128.41 (**CH**_{Ar}), 127.63 (**CH**_{Ar}), 126.54 (**CH**_{Ar}), 81.08 (C=**C-H)**, 80.82 (**C**_{q,Brücke}), 75.14 (p, *J =* 12.0 Hz, **CH₂**-SF₅), 69.55 (C≡**C**-H), 61.48 (**O-CH₂**), 19.99 (**CH₂-** C≡C).

**¹⁹F NMR** (471 MHz, CDCl₃) δ = 84.63 (p, J = 147.4 Hz, 1 **Fₐₓ**), 72.52 (dt, J=148.4 Hz, 7.4 Hz, 4 **F_{eq}**).

HR-EI-MS m/z (calc.) = 376.0920 [M'+]; m/z (found) = 376.0921 [M˙⁺] (C₁₈H₁₇F₅OS). Die Masse wurde aus der nicht trennbaren Isomerenmischung bestimmt.

### Beispiel 6: (2-(Cyclopentyloxy)-2,2-diphenylethyl)-pentafluor-λ⁶-sulfan

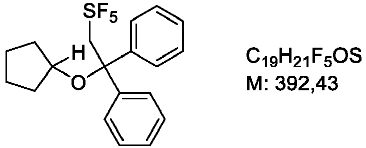

In einem Glasrohr mit Young-Hahn wurden 35.3 µl (36,1 mg, 0,200 mmol, 1,00 eq.) 1,1-Diphenylethylen sowie 181 µl (2,00 mmol, 10,0 eq.) Cyclopentanol in 1,0 ml filtriertem Sureseal Acetonitril gelöst. Anschließend wurden 5,5 mg (0,02 mmol, 10 mol%) N-Phenylphenothiazin und 20 µl (0,20 mmol, 10 mol%) Triethylboran (1M in Hexan) zur Reaktionsmischung gegeben. Die Atmosphäre wurde gegen Schwefelhexafluorid getauscht und die Reaktionsmischung wurde für 22 h bei 20°C unter Belichtung bei 368 nm gerührt. Anschließend wurde das Produkt durch ¹⁹ F-NMR-Spektroskopie quantifiziert Das Produkt wurde durch Säulenchromatographie gereinigt und als farbloses viskoses Öl erhalten (31%).

**¹H NMR** (500 MHz, CDCl3) δ = 7.28 - 7.20 (m, 10H, C**H**_{Ar}), , 4.69 (p, J=7.8, 2H, **CH₂**-SF₅), 3.93 (p, J=6.1, 1H, **O-CH**), 1.66 - 1.54 (m, 2H, **CH**_{**2,**cProp}), 1.49 - 1.39 (m, **CH**_{**2,**cProp}).

**¹³C NMR** (126 MHz, CDCl3) δ 143.65 (**C**_{q,Ar}), 127.94 (**C**H_{Ar}), 127.83 (**CH**_{Ar}), 127.61 (**CH**_{Ar}), 81.15 (**C**_{q,Brücke}), 77.0 (p, *J* = 9.8 Hz, **CH₂**-SF₅), 76.41 (O-**CH**), 33.79 **(CH₂**), 23.54 (**CH₂)**.

**19F NMR** (471 MHz, CDCl3) δ = 86.70 - 84.30 (p, *J* =149.5 Hz, 1 **Fₐₓ**), 73.12 (dt, J=148.9, 7.4, 4 **F_{eq}**).

HR-EI-MS m/z (calc.) = 392.1233 [M'+]; m/z (found) = 392.1233 [M˙⁺] (C₁₉H₂₁OF₅S).

### Beispiel 7: 3-(-(1-(Pentafluor-λ⁶-sulfanyl)-2-phenylpropan-2-yl)-oxy)-propannitril

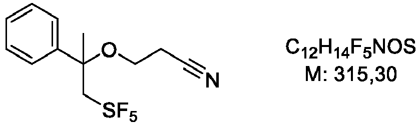

In einem Glasrohr mit Young-Hahn wurden 23,1 µL (21,0 mg, 0,178 mmol, 1,00 eq.) α-Methylstyrol sowie 205 µl (213 mg, 3,00 mmol, 16,9 eq.) 3-Hydroxypropionitril in 1 ml Sureseal^{®} Acetonitril (filtriert) gelöst. Anschließend wurden 5,5 mg (0,2 mmol, 11 mol%) N-Phenylphenothiazin zugegeben sowie 20 µl (0,20 mmol, 11 mol%) Triethylboran. Die Atmosphäre wurde gegen Schwefelhexafluorid getauscht und die Reaktionsmischung wurde für 22 h bei 20°C unter Belichtung bei 368 nm gerührt. Das Reaktionsprodukt wurde durch NMR-Spektroskopie quantifiziert und durch Säulenchromatographie aufgereinigt (Silica, 20% DCM in Hexan). Das Produkt wurde als farbloser Feststoff (29%) erhalten.

**¹H NMR** (500 MHz, CDCl3) δ = 7.46 (d, J=7.7, 2H, C**H**_{Ar}), 7.41 (t, J=7.6, 2H, C**H**_{Ar}), 7.35 (t, J=7.1, 1H, C**H**_{Ar}), 3.99 (dt, J=14.2, 8.6, 1H, **CH₂**SF₅), 3.82 (dt, J=14.2, 8.4, **CH₂**SF₅), 3.54 (dt, J=8.8, 6.1, 1H, O**-CH₂**), 3.36 - 3.22 (m, 1H, O**-CH₂**), 2.68 - 2.46 (m, 2H, **CH₂**), 1.91 (s, 3H, **CH₃**).

**¹³C NMR** (126 MHz, CDCl3) δ = 141.03 (**C**_{q,Ar}), 129.06 (**CH**_{Ar}), 128.77 (**CH**_{Ar}), 126.43, 117.78 (**CN**), 80.89 (p, *J* = 11.0 Hz, **CH₂**-SF₅), 78.59 (C_{q,Brücke}), 57.76 (O**-CH₂)**, 19.24 (**CH₂**-CN), 21.32 (**CH₃**).

**¹⁹F NMR** (471 MHz, CDCI3) δ = 86.16 - 82.37 (m, 1 **Fₐₓ**), 70.13 (dt, *J* = 147.0, 9.1, 4 **F_{eq}**).

**HR-EI-MS** m/z (calc.) = 315.0716 [M^{·+}]; m/z (found) = 315.0715 [M˙⁺] (C₁₂H₁₄F₅NOS).

### Beispiel 8: (2-(Allyloxy)-2,2-diphenylethyl)-pentafluor-λ⁶-sulfan

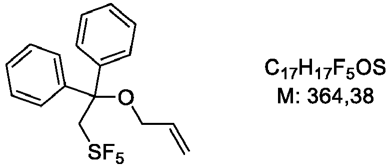

In einem Glasrohr mit Young-Hahn wurden 35,3 µl (36,1 mg, 0,200 mmol, 1,00 eq.) 1,1-Diphenylethylen sowie 136,5 µL (1,00 mmol, 10,0 eq.) Allylalkohol in 1 ml Sureseal^{®} Acetonitril (filtriert) gelöst. Anschließend wurden 5,5 mg (0,020 mmol, 10 mol%) N-Phenylphenothiazin zugegeben sowie 20 µl (0,020 mmol, 10 mol%) Triethylboran. Die Atmosphäre wurde gegen Schwefelhexafluorid getauscht und die Reaktionsmischung wurde für 21 h bei 20°C unter Belichtung bei 368 nm gerührt. Das Reaktionsprodukt wurde durch NMR-Spektroskopie quantifiziert und durch Säulenchromatographie aufgereinigt (Silica, 10% DCM in Hexan). Das Produkt wurde als hochviskoses Öl (32%) erhalten.

**¹H NMR** (500 MHz, Chloroform-*d*) δ 7.39 - 7.19 (m, 10H, C**H**_{Ar}), 5.92 (ddd, *J* = 17.3, 10.2, 5.0 Hz, 1H, C=**CH₂**), 5.39 (dq, *J* = 17.3, 1.9 Hz, 1H, C=**CH₂**), 5.18 (dq, *J* = 10.5, 1.7 Hz, 1H, **CH**=CH₂), 4.73 (p, *J* = 7.9 Hz, 2H, **CH₂**-SF₅), 3.76 (dt, *J* = 5.0, 1.7 Hz, 2H, O-**CH₂**).

**¹³C NMR** (126 MHz, Chloroform-*d*) δ 142.81 (**C**_{q,Ar}), 134.22 (**CH**=CH₂), 128.38 (**CH**_{Ar}), 127.63 (**CH**_{Ar}), 126.69 (**CH**_{Ar}), 116.27 (CH=**CH₂**), 81.06 (**C**_{q,Brücke}), 75.69 (p, *J* = 11.2 Hz, **CH₂**-SF₅), 64.50 (O-**CH₂**).

**¹⁹F NMR** (471 MHz, Chloroform-*d*) δ 84.32 (p, *J* = 148.4, 1 **Fₐₓ**), 72.06 (dt, *J* = 148.6, 8.1 Hz, 4 **F_{eq}**).

**HR-EI-MS** m/z (calc.) = 364.0920 [M˙⁺]; m/z (found) = 364.0919 [M˙⁺] (C₁₇H₁₇F₅OS).

### Beispiel 9: Pentafluor-(2-(pent-3-in-1-yloxy)-2-phenylpropyl)-λ⁶-sulfan

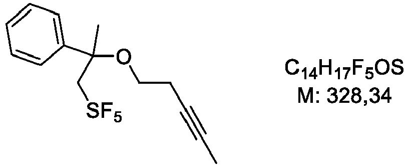

In einem Glasrohr mit Young-Hahn wurden 26,0 µl (23,6 mg, 0,20 mmol, 1,00 eq.) α-Methylstyrol sowie 186 µl (2,00 mmol, 10,0 eq.) 3-Pentin-1-ol in 1,0 ml Sureseal^{®} Acetonitril (filtriert) gelöst. Anschließend wurden 5,5 mg (0,020 mmol, 10 mol%) N-Phenylphenothiazin zugegeben sowie 20 µl (0,20 mmol, 20 mol%) Triethylboran (1M in Hexan) zur Reaktionsmischung gegeben. Die Reaktionsmischung wurde durch drei *freeze-pump-thaw-Zyklen* entgast und die Atmosphäre wurde gegen Schwefelhexafluorid getauscht. Die Reaktionsmischung wurde anschließend für 22 h bei 20°C unter Belichtung bei 368 nm gerührt. Das Produkt wurde durch Säulenchromatographie (Silica, Hexan (100%) bis DCM (5%) in Hexan) gereinigt und als farbloses viskoses Öl (33%) erhalten (R_{f} in Hexan/DCM (1:1) ca. 0.7).

**¹H NMR** (500 MHz, Chloroform-*d*) δ 7.45 (d, *J* = 7.1 Hz, 2H, **CH**_{Ar}), 7.38 (t, *J* = 7.6 Hz, 2H, **CH**_{Ar}), 7.34 - 7.29 (m, 1H, **CH**_{Ar}), 3.95 (dp, *J* = 14.1, 8.7 Hz, 1H, **CH₂**-SF₅), 3.82 (dp, *J* = 14.2, 8.5 Hz, 1H, **CH₂**-SF₅), 3.40 (dt, *J* = 7.5 Hz, 1H, O-**CH**_{**2**,**dia**}), 3.16 (dt, *J* = 7.7 Hz, 2H, O-**CH**_{**2,dia**,}), 2.38 (tq, *J* = 7.3, 2.6 Hz, 2H, **CH₂**), 1.86 (s, 1H, **CH₃**), 1.76 (t, *J* = 2.6 Hz, 1H, **CH₃**).

**¹³C NMR** (126 MHz, CDCl₃) δ 142.16 (**C**_{q,Ar}), 128.71 (**CH**_{Ar}), 128.26 (**CH**_{Ar}), 126.52 (**CH**_{Ar}), 81.22 (p, J_{CF} = 9.9 Hz, **CH₂**-SF₅), 78.02 (C≡C), 77.37 (C_{q,Brücke}), 75.90 (C≡C), 61.71 (O**-CH₂)**, 21.60 (C-**CH₃**), 20.51 (**CH₂**-C≡C), 3.56 (C≡C-**CH₃**).

**¹⁹F NMR** (471 MHz, Chloroform-d) δ 85.89 - 84.15 (m, 1 **Fₐₓ**), 70.12 (dt, *J* = 146.7, 8.8 Hz, 4 **F_{eq}**).

**HR-EI-MS** m/z (calc.) = 328.0920 [M^{·+}]; m/z (found) = 328.0923 [M˙⁺] (C₁₄H₁₇O₁F₅S).

### Beispiel 10: (2-(Allyloxy)-2-phenylpropyl)-pentafluor-λ⁶-sulfan

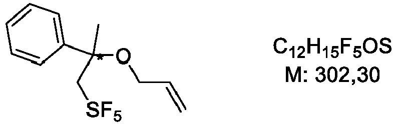

In einem Glasrohr mit Young-Hahn wurden 26,0 µl (23,6 mg, 0,20 mmol, 1,00 eq.) α-Methylstyrol sowie 68,3 µl Allylalkohol (2,65 mmol, 13,3 eq.) in 1 ml Sureseal^{®} Acetonitril (filtriert) gelöst. Anschließend wurden 1,4 mg (0,050 mmol, 5 mol%) N-Phenylphenothiazin zugegeben und 10 µl (0,10 mmol, 10 mol%) Triethylboran (1M in Hexan) zur Reaktionsmischung gegeben. Die Reaktionsmischung wurde durch drei *freeze-pump-thaw-Zyklen* entgast und die Atmosphäre wurde gegen Schwefelhexafluorid getauscht. Die Reaktionsmischung wurde anschließend für 22 h bei 20°C unter Belichtung bei 368 nm gerührt. Das Produkt wurde durch Säulenchromatographie (Silica, Hexan bis 5 (% DCM in Hexan) gereinigt und als farbloses viskoses Öl (20%) erhalten.

**¹H NMR** (500 MHz, Chloroform-*d*) δ 7.36 (d, *J* = 7.6 Hz, 2H, C**H**_{Ar}), 7.31 (t, *J* = 7.6 Hz, 2H, C**H**_{Ar}), 7.25 (t, *J* = 7.2 Hz, 1H, C**H**_{Ar}), 5.81 (ddt, *J* = 17.3, 10.1, 4.9 Hz, 1H, **HC**=CH₂), 5.26 (dq, *J* = 17.2, 1.9 Hz, 1H, HC=**CH₂**), 5.08 (dt, *J* = 10.5, 1.8 Hz, 1H, HC=**CH₂**), 3.97 (dq, J=17.6, 8.7, 1H, **CH_{2,dia}**-SF₅), 3.85 (m, 2H, **CH_{2,dia}**-SF₅ und **CH₂, _{dia}**-CH=CH₂), 3.64 (dd, J=12.8, 4.7, 1H, **CH**_{**2**,**dia**}-CH=CH2), 1.81 (s, 3H, **CH₃**).

**¹³C NMR** (126 MHz, Chloroform-*d*) δ 142.15 (**C**_{q,Ar}), 134.71 (**CH**=CH₂), 128.78 (**CH**_{Ar}), 128.30 (**CH**_{Ar}), 126.47 (**CH**_{Ar}), 115.89 (CH=**CH₂**), 81.34 (p, *J* = 10.1 Hz, **CH₂**-SF₅), 78.27 (C_{q,Brücke}), 63.78 (O**-CH₂)**, 21.71 (**CH₃**).

**¹⁹F NMR** (471 MHz, Chloroform-*d*) δ 86.29 - 83.42 (m, 1 **Fₐₓ**), 70.15 (dt, *J* = 146.7, 8.9 Hz, 4 **F_{eq}**).

**EI-MS** m/z (calc.) = 302.0 [M^{·+}]; m/z (found) = 301.1 [M-H^{·+}], 287.0 [M-Me^{·+}] (C₁₁H₁₂OFS₅).

### Beispiel 11: (2-(Cyclopentyloxy)-2-phenylpropyl)pentafluoro-λ⁶-sulfan

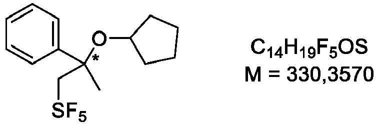

In einem Glasrohr mit Young-Hahn wurden 23,1 µl (21,0 mg, 0,178 mmol, 1,00 eq.) α-Methylstyrol sowie 181 µl (172 mg, 2,00 mmol, 11,2 eq) Cyclopentanol in 1 ml filtriertem Sureseal^{®} Acetonitril gelöst. Anschließend wurden 5,5 mg (0,02 mmol, 11 mol%) N-Phenylphenothiazin zugegeben und 20 µl (0,20 mmol, 11 mol%) Triethylboran (1M in Hexan) zur Reaktionsmischung gegeben. Die Reaktionsmischung wurde durch drei *freeze-pump-thaw-Zyklen* entgast und die Atmosphäre wurde gegen Schwefelhexafluorid getauscht. Die Reaktionsmischung wurde für 22 h bei 20°C unter Belichtung bei 368 nm gerührt. Anschließend wurde das Produkt durch ¹⁹F-NMR-Spektroskopie quantifiziert und durch Säulenchromatographie (Silica, Hexan, Rf (Hexan) = 0.4) gereinigt und als farbloses viskoses Öl erhalten (22%).

**¹H NMR** (500 MHz, CDCl₃) δ = 7.46 (d, J=7.7, 2H, C**H**_{Ar}), 7.37 (t, J=7.6, 2H, C**H**_{Ar}), 7.31 (t, J=7.3, 1H, C**H**Ar), 3.91 (dp, J=14.1, 8.8, 1H, **CH_{2,dia}**-SFs), 3.82 - 3.64 (m, 2H, **CH_{2,dia}**-SF₅ und O-**CH**(CH₃)₂), 1.87 (s, 3H, **CH₃**), 1.77 - 1.31 (m, 8H, 4 × **CH_{2,cProp,dia}**).

**¹³C NMR** (126 MHz, CDCl₃) δ = 143.66 (**C**_{q,Ar}), 128.52 (**CH_{Ar}**), 128.06 (**CH_{Ar}**), 126.87 (**CH_{Ar}**), 82.01 (p, J = 9.4 Hz, **CH₂**-SF₅), 78.29 (**C**_{q,Brücke}), 75.76 (O-**CH**(CH₃)₂), 34.65 (**CH**_{**2**,dia}), 34.22 (**CH**_{**2**,dia}), 23.96 (**CH**_{**2**,dia}), 23.44 (**CH**_{**2**,dia}), 22.58 (**CH₃**).

**¹⁹F NMR** (471 MHz, CDCl3) δ = 86.08 (p, J=147.1, 1F,1 **Fₐₓ**), 70.66 (dt, J=146.2, 9.3, 4 **Fₐₓ**).

**HR-EI-MS** m/z (calc.) = 330.3570 [M'+]; m/z (found) = 245.0423 [M-cycPentO^{·+}] (C₉H₁₀F₅S).

### Beispiel 12: Pentafluor-(2-(pent-4-in-1-yloxy)-2-phenylpropyl)-λ⁶-sulfan

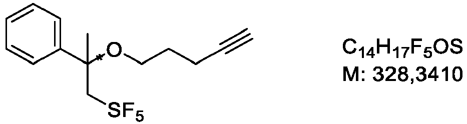

In einem Glasrohr mit Young-Hahn wurden 26,0 µl (23,6 mg, 0,20 mmol, 2,00 eq.) α-Methylstyrol sowie 187 µl (168 mg, 1,00 mmol, 10,0 eq.) 4-Pentin-1-ol in 1 ml Sureseal^{®} Acetonitril (filtriert) gelöst. Anschließend wurden 5,5 mg (0,020 mmol, 10 mol%) N-Phenylphenothiazin und 20 µl (0,020 mmol, 10 mol%) Triethylboran (1M in Hexan) zur Reaktionsmischung gegeben. Die Reaktionsmischung wurde durch drei *freeze-pump-thaw-Zyklen* entgast und die Atmosphäre wurde gegen Schwefelhexa-fluorid getauscht. Die Reaktionsmischung wurde für 22 h bei 20°C unter Belichtung bei 368 nm gerührt. Anschließend wurde das Produkt durch ¹⁹F-NMR-Spektroskopie quantifiziert und durch Säulenchromatographie (Silica, Hexan bis 5% DCM in Hexan) gereinigt und als farbloses viskoses Öl (17%) erhalten (R_{f} in Hexan/DCM (1:1) ca. 0.7).

**¹H** NMR (500 MHz, Chloroform-*d*) δ 7.42 (d, *J* = 7.8 Hz, 2H, **CH_{Ar}**), 7.38 (dd, *J* = 8.3, 6.7 Hz, 2H, **CH_{Ar}**), 7.34 - 7.29 (m, 1H, **CH_{Ar}**), 3.92 (dq, J=17.5, 8.6, 1H, CH2,dia-SFs), 3.78 (dq, J=14.9, 8.3, 1H, **CH_{2,dia}**-SF₅), 3.41 (q, J=7.3, 1H, O-**CH_{2,dia}**), 3.17 (q, J=6.3, 1H, O-**CH_{2,dia}**), 2.34 (t, J=7.4, 2H, **CH₂**-C≡CH), 1.92 (t, *J* = 2.5 Hz, 1H, C≡C-**H**), 1.87 (s, **CH₃**), 1.84 - 1.69 (m, 2H, -CH₂₋**CH₂**-CH₂).

**¹³C** NMR (126 MHz, CDCl3) δ 142.36 (**C**_{q,Ar}), 128.75 (**CH**_{Ar}), 128.21 (**CH**_{Ar}), 126.45 (**CH**_{Ar}), 84.12 (CEC), 81.34 (p, *J* = 9.5 Hz, **CH₂**-SF₅), 77.78 (**C**_{q,Brücke}), 68.58 (C=CH), 60.65 (O**-CH₂)**, 29.03 (CH₂), 21.56 (**CH₃**), 15.33 (CH₂-**CH₂**-CH₂).

**¹⁹F-NMR** (471 MHz, CDCl3) δ = 85.29 (p, J=147.1, 1F, **Fₐₓ**), 70.58 (dt, J = 146.7, 8.3 Hz, 4F, **F_{eq}**).

**HR-EI-MS** m/z (calc.) = 328.0920 [M'+]; m/z (found) = 328.0922 [M^{·+}] (C₁₄H₁₇OF₅S). Aus der Isomerenmischung bestimmt.

### Beispiel 13: 2-((Pentafluor-λ⁶-sulfanyl)-methyl)-2-phenyltetrahydrofuran

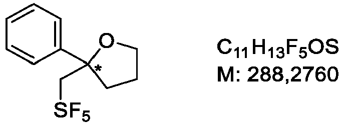

In einem Glasrohr mit Young-Hahn wurden 50,0 µl (53,5 mg, 0,330 mmol, 1,00 eq.) 4-Phenylpent-4-en-1-ol in 1 ml filtriertem Sureseal^{®} Acetonitril gelöst. Anschließend wurden 5,5 mg (0,02 mmol, 6,1 mol%) N-Phenylphenothiazin zugegeben und 20 µl (0,02 mmol, 6,1 mol%) Triethylboran (1M in Hexan) zur Reaktionsmischung gegeben. Die Reaktionsmischung wurde durch drei *freeze-pump-thaw-Zyklen* entgast und die Atmosphäre wurde gegen Schwefelhexafluorid getauscht. Die Reaktionsmischung wurde für 22 h bei 20°C unter Belichtung bei 368 nm gerührt. Anschließend wurde das Produkt durch ¹⁹F-NMR-Spektroskopie quantifiziert und das Produkt durch Säulenchromatographie (Silica, 5% DCM in Hexan, Rf = 0.3) gereinigt und als farbloses viskoses Öl erhalten (26%). Das Produkt wurde aufgrund seiner Flüchtigkeit in Anwesenheit von Resten von Hexan charakterisiert.

**¹H NMR** (500 MHz, CDCl3) δ = 7.40 (d, J=7.4, 2H, **CH**_{Ar}), 7.35 (dd, J=8.6, 6.7, 2H, **CH**_{Ar}), 7.28 (d, J=7.1, 1H, **CH**_{Ar}), 4.24 - 3.92 (m, 4H, **CH₂**SF₅ und O**-CH₂)**, 2.29 (dd, J=8.1, 6.5, 2H, CH₂-**CH₂-**CH₂), 2.07 - 1.90 (m, 1H, C_{q}-**CH**_{**2**,dia}), 1.87 - 1.78 (m, 1H, C_{q}-**CH**_{**2**,dia}).

**¹³C NMR** (126 MHz, CDCl3) δ = 143.69 (**C**_{q,Ar}), 128.40 (**CH**_{Ar}), 127.48 (**CH**_{Ar}), 125.44 (**C**H_{Ar}), 85.04 (**C**_{q,Brücke})78.93 (q, *J* = 10.0, **CH₂**-SF₅), 68.65 (O**-CH₂)**, 38.75 (**CH₂**), 25.14 (C_{q}-**CH₂**).

**¹⁹F NMR** (471 MHz, CDCl3) δ = 84.51 (p, J=148 Hz, 1F,**Fₐₓ**), 69.80 (dt, J=147, 8.8, 4F, **F_{eq}**).

**HR-EI-MS** m/z (calc.) = 288.0796 [M˙⁺]; m/z (found) = 288.0794 [M˙⁺] (C₁₁H₁₃F₅OS).

Allgemeine Reaktionsvorschrift A zur Herstellung weiterer alpha-Alkoxypentafluorsulfanylverbindungen:
In einem Glasrohr mit Young-Hahn wurden 0,20 mmol (1,00 eq.) des Alkens zu 1 ml Acetonitril (Surseal-Grad über Molekularsieben gelagert, gefiltert) zugegeben. Dann wurden 2,00 mmol (10,0 eq.) des Alkohols sowie 5,50 mg (0,02 mmol, 10 mol%) des Photoredox-Katalysators hinzugefügt. Schließlich wurden 20 µl (0,02 mmol, 10 mol%) BEt₃ (1,0 M in Hexan) dem Reaktionsgemisch zugegeben. Das Reaktionsgemisch wurde durch drei *freeze-pump-thaw-Zyklen* entgast und mit Schwefelhexafluorid (2,8 bar, 15 Äquivalente) nachgefüllt. Das Reaktionsgemisch wurde bei Raumtemperatur unter Bestrahlung bei 368 nm durch eine Hochleistungs-LED bei 20 °C für 22 h gerührt. Die Ausbeute wurde durch ¹⁹F-NMR-Spektroskopie mit α,α,α-Trifluorotoluol als internem Standard bestimmt. Die Verbindungen wurden mittels Säulenchromatographie mit Siliziumdioxid als stationäre Phase und Dichlormethan in Hexan bzw. in Cyclohexan als Elutionsmittel gereinigt.

### Beispiel 14: Pentafluor-(2-((2-methylallyl)-oxy)-2,2-diphenylethyl)-λ⁶-sulfan

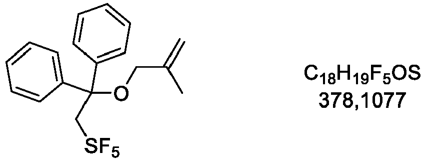

Die Verbindung wurde nach der allgemeinen Reaktionsvorschrift A hergestellt. In einem Glasrohr mit Young-Hahn wurden 35,3 µl (0,200 mmol, 1,00 eq.) Diphenylethylen sowie 168 µl (144 mg, 2,00 mmol, 10,0 eq.) 2-Methyl-2-propen-1-ol eingesetzt. Die Verbindung wurde durch Säulenchromatographie gereinigt (Silica, 5% DCM in Hexan, TLC: Rf = 0,3). Die Ausbeute des Produkts wurde durch die Analyse des Rohreaktionsgemischs mittels ¹⁹F-NMR-Spektroskopie (26%) bestimmt. Das Produkt wurde als farbloser Feststoff erhalten.

**¹H NMR** (500 MHz, Chloroform-*d*) δ 7.38 - 7.28 (m, 8H), 7.27 - 7.21 (m, 2H), 5.21 - 5.01 (m, 1H), 4.90 (p, *J* = 1.3 Hz, 1H), 4.73 (p, *J* = 7.8 Hz, 2H), 3.65 (s, 2H), 1.75 (t, *J* = 1.2 Hz, 3H).

**¹³C NMR** (126 MHz, Chloroform-*d*) δ 142.86 (**C**_{q,Ar}), 141.79 (**C**_{q,vinyl}), 128.37 (**C**H_{Ar}), 127.62 (**C**H_{Ar}), 126.76 (**C**H_{Ar}), 111.51 (**C**H_{2,vinyl}), 81.02 (**C**_{q,Bridge}), 75.91 (**C**H₂SF₅), 67.08 (**C**H₂O), , 19.99 (**C**H**₃**).

**¹⁹F NMR** (471 MHz, Chloroform-*d*) δ 84.31 (p, *J* = 150 Hz, 1F, **Fₐₓ**), 72.07 (dt, *J* = 149, 8.1 Hz, 4F, **F_{eq}**).

**HR-EI-MS** m/z (calc.) = 378.1077 [M˙⁺]; m/z (found) = 378.1079 [M˙⁺] (C₁₈H₁₉F₅OS).

### Beispiel 15: 3-(2-(Pentafluor-λ⁶-sulfanyl)-1,1-diphenylethoxy)-propionnitril

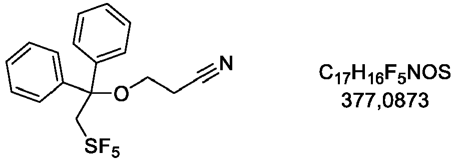

Die Verbindung wurde nach der allgemeinen Reaktionsvorschrift A hergestellt. In einem Glasrohr mit Young-Hahn wurden 35,3 µl (0,200 mmol, 1,00 eq.) Diphenylethylen 2 sowie 137 µl (142 mg, 2,00 mmol, 10,0 eq.) 3-Hydroxypropionitril eingesetzt. Die Verbindung wurde durch Säulenchromatographie (Siliziumdioxid, 50% DCM in Hexan) gereinigt. Die Ausbeute des Produkts wurde durch die Analyse des Rohreaktionsgemischs mittels 19F-NMR-Spektroskopie (29%) bestimmt. Das Produkt wurde als farblose Nadeln erhalten.

**¹H NMR** (500 MHz, Chloroform-*d*) δ 7.33 (m, 8H, **CH_{Ar}**), 7.27 (m, 2H, **CH_{Ar}**), 4.73 (p, *J* = 7.7 Hz, 2H, **CH₂**SF₅), 3.46 (t, *J* = 6.4 Hz, 2H, O**CH₂**), 2.66 (t, *J* = 6.4 Hz, 2H, **CH₂**CN).

**¹³C NMR** (126 MHz, Chloroform-*d*) δ 142.11 (**C_{q,Ar}**), 128.64 (**CH_{Ar}**), 127.97(**CH_{Ar}**), 126.44 (**CH_{Ar}**), 117.58 (**C≡N**), 81.30 (**C**_{q,bridge}), 74.95 (p, *J* = 11.2 Hz, **CH₂**SF₅), 58.28 (O**-CH₂)**, 19.02 (**CH₂**CN).

**¹⁹F NMR** (471 MHz, Chloroform-*d*) δ 83.91 (p, *J* = 148 Hz, 1F, **Fₐₓ**), 72.03 (dt, *J* = 149, 8.0 Hz, 4F, **F_{eq}**).

**HR-EI-MS** m/z (calc.) = 377.0873 [M˙⁺]; m/z (found) = 377.0873 [M˙⁺] (C₁₇H₁₆F₅NOS).

### Beispiel 16: Pentafluor-(2-isopropoxy-2,2-diphenylethyl)-λ⁶-sulfan

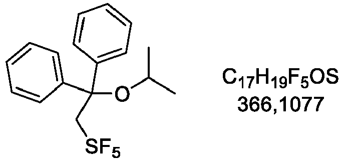

Die Verbindung wurde nach der allgemeinen Reaktionsvorschrift A hergestellt. In einem Reaktionsgefäß vom Typ Young wurden 35,3 µl (0,200 mmol, 1,00 eq.) Diphenylethylen 2 sowie 154 µl (120 mg, 2,00 mmol, 10,0 eq.) *i*-PrOH eingesetzt. Die Verbindung wurde durch Säulenchromatographie (Siliziumdioxid, 5% DCM in Hexan) gereinigt. Die Ausbeute des Produkts wurde durch die Analyse des Rohreaktionsgemischs mittels ¹⁹F-NMR-Spektroskopie (20%) bestimmt. Das Produkt wurde als farbloses Öl gewonnen.

*Scale-up:* Die Reaktion wurde ebenfalls im 5-fachen Maßstab (1 eq. = 1 mmol) nach der allgemeinen Reaktionsvorschrift A mit 5,5 bar SF₆ (6,0 eq.) durchgeführt. Die Ausbeute betrug 29 %, bestimmt durch NMR-Spektroskopie.

**¹H NMR** (500 MHz, Chloroform-*d*) δ 7.50 (d, *J* = 7.7 Hz, 2H, C**H**_{Ar}), 7.36 (t, *J* = 7.4 Hz, 2H, C**H**_{Ar}), 7.31 (t, *J* = 7.3 Hz, 1H, C**H**_{Ar}), 4.00 - 3.89 (m, 1H, **CH₂**SF₅), 3.89 - 3.75 (m, 1H,**CH₂**SF₅), 3.55 (h, *J* = 6.1 Hz, 1H,C**H**iPr), 1.91 (s, 3H, **CH₃**), 1.15 (d, *J* = 6.1 Hz, 3H, CH**CH₃**), 0.93 (d, *J* = 6.1 Hz,3H, CH**CH₃**).

**¹³C NMR** (126 MHz, Chloroform-*d*) δ 143.06 (**C**_{q,Ar}), 128.32 (**CH**_{Ar}), 128.19 (**CH**_{Ar}, 127.05 (**CH**_{Ar}), 77.88 (**C**_{q,Bridge}) , 66.09 (**CH₂**-SF5) , 24.88 (**CH₃**-iPr) , 24.36 (**CH₃**-iPr), 22.00 (**CH₃**).

**¹⁹F NMR** (471 MHz, Chloroform-*d*) δ 85.77 (p, *J* = 151 Hz, 1F, **Fₐₓ**), 72.69 (dt, *J* = 149, 8.2 Hz, 4F, **F_{eq}**).

**HR-EI-MS** m/z (calc.) = 366.1077 [M˙⁺]; m/z (found) = 366.1076 [M˙⁺] (C₁₇H₁₉OF₅S).

### Beispiel 17: Pentafluor-(2-(penta-3,4-dien-1-yloxy)-2,2-diphenylethyl)-λ⁶-sulfan

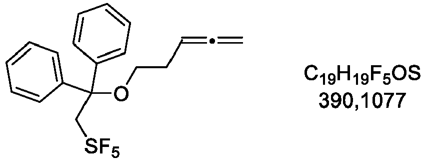

Die Verbindung wurde nach einer modifizierten Reaktionsvorschrift A hergestellt. In einem Glasrohr mit Young-Hahn wurden 26,0 µl (26,5 mg, 0,148 mmol, 1,00 eq.) Diphenylethylen sowie 188 µl (168 mg, 2,00 mmol, 13,6 eq.) 3,4-Pentadien-1-ol eingesetzt. Die Verbindung wurde durch Säulenchromatographie gereinigt (Siliziumdioxid, 5% DCM in Hexan, Rf = 0,3 in 10% DCM in Hexan). Die Ausbeute des Produkts wurde durch die Analyse des Rohreaktionsgemischs mittels ¹⁹F-NMR-Spektroskopie (19%) bestimmt. Das Produkt wurde als farbloses Öl gewonnen.

**¹H NMR** (500 MHz, Chloroform-*d*) δ 7.33 - 7.27 (m, 8H, **CH_{Ar}**), 7.23 (ddt, *J* = 6.3, 5.3, 2.6 Hz, 2H, **CH_{Ar}**), 5.16 (p, *J* = 7.0 Hz, 1H, CH₂**CH**=C=), 4.73 (p, *J* = 7.9 Hz, 2H, **CH₂**SF₅), 4.69 - 4.63 (m, 2H, CH₂CH=**CH₂**), 3.28 (t, *J* = 6.6 Hz, 2H, O**CH₂**), 2.44 - 2.24 (m, 2H, **CH₂**CH=C=).

**¹³C NMR** (126 MHz, Chloroform-*d*) δ 209.19 (**C**_{q,allene}), 143.26 (**C**_{q,Ar}), 128.32 (**C**H_{Ar}), 127.50 (**C**H_{Ar}), 126.62 (**C**H_{Ar}), 86.79 (C**C**H=CH₂), 75.26 (p, *J* = 10.7 Hz, CH₂SF₅), 74.93 (**C**H₂=C), 62.56 (O**C**H₂), 29.23 (OCH₂**C**H₂).

**¹⁹F NMR** (471 MHz, Chloroform-*d*) δ 84.45 (p, *J* = 149 , 1F, **Fₐₓ**), 72.10 (dt, *J* = 149.1, 8.0 Hz, 4F, **F_{eq}**).

**HR-EI-MS** m/z (calc.) = 263.1436 [M-SF₅ ^{·+}]; m/z (found) = 263.1435 [M-SF₅ ^{·+}] (C₁₉H₁₉O); m/z (calc.) = 249.1279 [M-CH₂SF₅^{·+}]; m/z (found) = 249.1278 [M-CH₂SF₅ ^{·+}] (C₁₈H₁₇O).

### Beispiel 18: Pentafluor-(2-methoxy-2-phenylpropyl)-λ⁶-sulfan

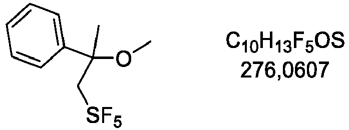

Die Verbindung wurde nach der allgemeinen Reaktionsvorschrift A hergestellt. In einem Glasrohr mit Young-Hahn wurden 26,0 µl (0,200 mmol, 1,00 eq.) α-Methylstyrol sowie 81,1 µl (64,1 mg, 2,00 mmol, 10,0 eq.) MeOH eingesetzt. Die Verbindung wurde durch Säulenchromatographie (Siliziumdioxid, Hexan bis 2% DCM in Hexan) gereinigt. Das Produkt wurde als flüchtiges Öl gewonnen. Die Ausbeute des Produkts wurde durch die Analyse des Rohreaktionsgemischs mittels ¹⁹F-NMR-Spektroskopie (37%) bestimmt.

**¹H NMR** (500 MHz, Chloroform-*d*) δ 7.45 - 7.36 (m, 4H, **CH**_{Ar}), 7.34 - 7.30 (m, 1H, **CH**Ar), 3.94 (dp, *J* = 14.2, 8.8 Hz, 1H, **CH_{2,dia}**SFs), 3.81 (dp, *J* = 14.2, 8.7 Hz, 1H, **CH_{2,dia}**SF₅), 3.12 (s, 3H, **OCH₃**), 1.85 (s, 3H, **CH₃**).

**¹³C NMR** (126 MHz, Chloroform-*d*) δ 142.02 (**C**_{q,Ar}), 128.78 (**CH**_{Ar}), 128.23 (**CH**_{Ar}), 126.54 (**CH**_{Ar}), 81.13 (p, *J* = 10.0 Hz, **CH_{2,dia}**-SF₅), 78.33 (p, *J* = 2.3 Hz, **C**_{q,bridge}), 50.64 (O**CH₃**), 21.13 (p, *J* = 2.2 Hz, **CH₃**).

**¹⁹F NMR** (471 MHz, Chloroform-*d*) δ 85.10 (p, *J* = 148 Hz, 1F, **Fₐₓ**), 70.01 (dt, *J* = 147, 8.8 Hz, 4F, **F_{eq}**).

HR-EI-MS m/z (calc.) = 276.0607 [M˙⁺]; m/z (found) = 276.0608 [M˙⁺] (C₁₀H₁₃OF₅S).

### Beispiel 19: Pentafluor-(2-((2-methylallyl)-oxy)-2-phenylpropyl)- λ⁶-sulfan

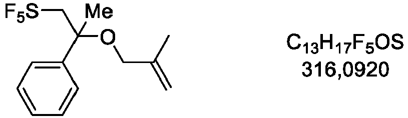

Die Verbindung wurde nach der allgemeinen Reaktionsvorschrift A hergestellt. In einem Glasrohr mit Young-Hahn wurden 26,0 µl (23,6 mg, 0,200 mmol, 1,00 eq.) α-Methylstyrol sowie 168 µl (144 mg, 2,00 mmol, 10,0 eq.) 2-Methyl-2-propen-1-ol eingesetzt. Die Verbindung wurde durch Säulenchromatographie (Siliziumdioxid, Cyclohexan) gereinigt. Die Ausbeute des Produkts wurde durch die Analyse des Rohreaktionsgemischs mittels ¹⁹F-NMR-Spektroskopie (31%) bestimmt. Das Produkt wurde als farbloses Öl gewonnen.

**¹H NMR** (500 MHz, Chloroform-*d*) δ 7.47 - 7.42 (m, 2H, C**H**_{Ar}), 7.41 - 7.36 (m, 2H, C**H**_{Ar}), 7.35 - 7.30 (m, 1H, C**H**_{Ar}), 5.04 (s, 1H, =**CHₐ**H_{b}), 4.87 (s, 1H, =**C**Hₐ**H_{b}**), 3.98 (dp, *J =* 14.2, 8.7 Hz, 1H, **CH_{2,dia}**-SF₅), 3.82 (dp, *J* = 14.1, 8.6 Hz, 1H, **CH_{2,dia}**-SF₅), 3.74 (d, *J* = 12.0 Hz, 1H, O**CH_{2,dia}**), 3.51 (d, *J* = 12.0 Hz, 1H, O**CH_{2,dia}**), 1.89 (s, 3H, **CH₃**), 1.72 (s, 3H, **CH₃**).

**¹³C NMR** (126 MHz, Chloroform-*d*) δ 142.15 (**C**=CH₂), 142.21 (**C**_{q,Ar}), 128.79 (**CH**_{Ar}), 128.29 (**CH**_{Ar}), 126.50 (**CH**_{Ar}), 111.09 (C=**CH₂**)., 81.46 (p, *J* = 10.0 Hz, **CH₂**SF₅), 78.20 (**C**_{q,bridge}), 66.51 (O**CH₂**), 21.55 (p, *J* = 2.1 Hz, C**CH₃**), 19.79 (C(C=CH₂)**CH₃**).

**¹⁹F NMR** (471 MHz, Chloroform-*d*) δ 85.10 (p, *J* = 147 Hz, 1F, **Fₐₓ**), 70.17 (dt, *J* = 147, 8.8 Hz, 4F, **F_{eq}**).

**HR-EI-MS** m/z (calc.) = 301.0686 [M-Me^{·+}]; m/z (found) = 301.0686 [M-Me^{·+}] (C₁₂H₁₄OF₅S); m/z (calc.) = 245.0423 [M-OMeAllyl^{·+}]; m/z (found) = 245.0422 [M-OMeAllyl^{·+}] (C₉H₁₀F₅S).

### Beispiel 20: Pentafluor(2-phenyl-2-(prop-2-in-1-yloxy)propyl)-λ⁶-sulfan

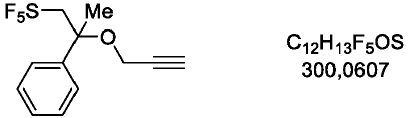

Die Verbindung wurde nach der allgemeinen Reaktionsvorschrift A hergestellt. In einem Glasrohr mit Young-Hahn wurden 26,0 µl (23,6 mg, 0,200 mmol, 1,00 eq.) α-Methylstyrol 1 sowie 35,5 µl (34,2 mg, 0,610 mmol, 3,04 eq.) Propargylakohol eingesetzt. Die Verbindung wurde durch IPLC vorgereinigt (Puriflash, RP-C18, 65% bis 72% MeCN in Wasser) und der produkthaltige Anteil wurde durch Säulenchromatographie gereinigt (Siliziumdioxid, Cyclohexan bis 10% DCM in Cyclohexan, Rf (50% DCM in Cyclohexanen = 0,76). Die Ausbeute des Produkts wurde durch die Analyse des Rohreaktionsgemischs mittels ¹⁹F-NMR-Spektroskopie (20%) bestimmt.

**¹H NMR** (500 MHz, Chloroform-*d*) δ 7.46 - 7.44 (m, 2H, **CH**_{Ar}), 7.40 (ddd, *J* = 7.8, 6.7, 1.3 Hz, 2H, **CH**_{Ar}), 7.37 - 7.31 (m, 1H, **CH**_{Ar}), 4.06 - 3.77 (m, 4H, **CH₂**SF5, O**CH₂**), 2.39 (t, *J* = 2.4 Hz, 1H, C≡**CH**), 1.93 (s, 3H, **CH₃**).

**¹³C NMR** (126 MHz, Chloroform-*d*) δ 141.15 (**C_{q,Ar}**), 128.93 (**CH**_{Ar}), 128.65 (**CH**_{Ar}), 126.64 (**CH**_{Ar}), 81.12 (p, *J* = 10.7 Hz, **CH₂**SF₅), 80.34 (**C**≡CH),, 79.30 (**C**_{q,bridge}), 73.94 (C≡**CH)**, 51.68 (O**CH₂**), 21.45 (**CH₃**).

**¹⁹F NMR** (471 MHz, Chloroform-*d*) δ 84.66 (p, *J* = 148 Hz, 1F, **Fₐₓ**), 70.16 (dt, *J* = 147, 8.7 Hz, 4F, **F_{eq}**).

**HR-EI-MS** m/z (calc.) = 300.0607 [M˙⁺]; m/z (found) = 300.0607 [M˙⁺]; (C₁₂H₁₃OF₅S).

### Beispiel 21: (2-(But-3-in-1-yloxy)-2-phenylpropyl)-pentafluor-λ⁶-sulfan

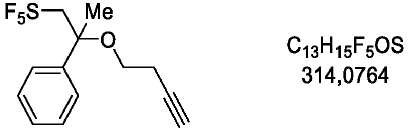

Die Verbindung wurde nach einer modifizierten Reaktionsvorschrift A hergestellt. In einem Glasrohr mit Young-Hahn wurden 26,0 µl (0,200 mmol, 1,00 eq.) α-Methylstyrol 1 sowie 45,4 µl (42,1 mg, 0,600 mmol, 3,00 eq.) 3-Butinol eingesetzt. Die Verbindung wurde durch IPLC vorgereinigt (Puriflash, RP-C18, 65% bis 73% MeCN in Wasser) und der produkthaltige Anteil wurde durch Säulenchromatographie gereinigt (Siliziumdioxid, Cyclohexan bis 10% DCM in Cyclohexan, Rf (50% DCM in Cyclohexanen = 0,61). Die Ausbeute des Produkts wurde durch die Analyse des Rohreaktionsgemischs mittels ¹⁹F-NMR-Spektroskopie (19%) bestimmt.

**¹H NMR** (500 MHz, Chloroform-*d*) δ 7.48 - 7.43 (m, 2H, **CH**_{Ar}), 7.42 - 7.36 (m, 2H, **CH**_{Ar}), 7.35 - 7.30 (m, 1H, **CH**_{Ar}), 3.96 (dp, *J* = 14.1, 8.8 Hz, 1H, **CH_{2,dia}**SFs), 3.82 (dp, *J* = 14.2, 8.6 Hz, 1H, **CH_{2,dia}**SF₅), 3.45 (dt, *J* = 8.5, 6.9 Hz, 1H, O**CH_{2,dia}**), 3.22 (dt, *J* = 8.5, 7.2 Hz, 1H, O**CH_{2,dia}**), 2.45 (td, *J* = 7.1, 2.7 Hz, 2H, **CH₂**), 1.96 (t, *J* = 2.7 Hz, 1H, C≡**C-H**), 1.88 (s, 3H, **CH₃**).

**¹³C NMR** (126 MHz, Chloroform-*d*) δ 141.98 (**C**_{q,Ar}), 128.78 (**CH**_{,Ar}), 128.35 (**CH**_{,Ar}), 126.50 (**CH**,_{Ar}), 81.30 (**C**≡CH)" 81.16 (p, *J* = 10.2 Hz, **CH₂**SF₅), 78.10 (p, *J* = 2.0 Hz, **C**_{q,bridge}), 69.47 (C≡**CH**), 61.06 (O**CH₂)**, 21.56 (p, *J* = 1.9 Hz, C**CH₃**), 20.26 (CH₂**CH₂**).

**¹⁹F NMR** (471 MHz, Chloroform-*d*) δ 85.01 (p, *J* = 147 Hz, 1F, **Fₐₓ**), 70.14 (dt, *J* = 147, 8.7 Hz, 4F, **F_{eq}**).

**HR-EI-MS** m/z (calc.) = 314.0764 [M˙⁺]; m/z (found) = 314.0766 [M˙⁺]; (C₁₃H₁₅OF₅S).

### Beispiel 22: (E)-7-(hex-5-in-1-yloxy)-5-((pentafluor-λ⁶-sulfanyl)methylen)-7,7-diphenylheptan-1-ol

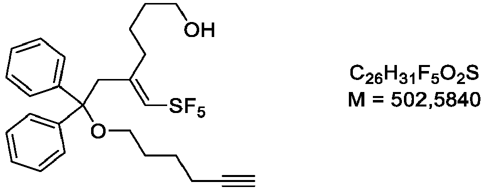

Die Verbindung wurde nach der allgemeinen Reaktionsvorschrift A hergestellt. In einem Glasrohr mit Young-Hahn wurden 35,3 µl (36,1 mg, 0,200 mmol, 1,00 eq.) Diphenylethylen sowie 441 µl (393 mg, 2,00 mmol, 20,0 eq.) 5-Hexin-1-ol eingesetzt. Die Verbindung wurde mittels Säulenchromatographie-System (C18, 65% MeCN in Wasser bis 71% MeCN in Wasser) vorgereinigt und die Produktfraktion mittels Säulenchromatographie (Siliziumdioxid, Hexan (0,1% NEts) bis DCM) gereinigt. Die Ausbeute des Produkts wurde durch die Analyse des Rohreaktionsgemischs mittels ¹⁹F-NMR-Spektroskopie (32%) bestimmt. Das Produkt wurde als hochviskoses gelbliches Öl gewonnen.

**¹H NMR** (500 MHz, Chloroform-*d*) δ 7.30 (m, 8H), 7.26 - 7.20 (m, 2H), 6.07 (p, *J* = 9.3 Hz, 1H), 3.58 (t, *J* = 6.2 Hz, 2H), 3.16 - 3.02 (m, 4H), 2.18 (td, *J* = 6.9, 3.2 Hz, 2H), 2.00 (t, *J* = 8.2 Hz, 2H), 1.94 (t, *J* = 2.6 Hz, 1H), 1.73 - 1.57 (m, 4H), 1.50 - 1.34 (m, 4H).

**¹³C NMR** (126 MHz, Chloroform-*d*) δ 144.57 (p, *J* = 4.7 Hz, **C**_{q}=CHSF₅), 143.95 (**C**_{q,Ar}), 139.00 (t, *J* = 17.1 Hz, **C**_{q}=**CH**SF₅), 128.18 (**CH**_{Ar}), 127.40 (**CH**_{Ar}), 84.38 (**C≡**CH), 82.54 (C_{q,bridge}), 68.63 (C**≡CH**), 62.50 (O**CH₂**), 62.20 (O**CH₂**), 40.81 (C_{q,asymm}**CH₂**C_{q}), 32.76 (**CH**_{**2,**int}), 32.39 (SF₅CH=C_{q}**CH₂**CH₂), 28.94 (**CH**_{**2,**int}), 25.42 (**CH**_{**2,**int}), 24.12 (**CH**_{**2,**int}), 18.37 (**CH₂**C≡CH).

**¹⁹F NMR** (471 MHz, Chloroform-*d*) δ 86.12 (p, *J* = 150 Hz, 1F, **Fₐₓ**), 65.56 (dd, *J* = 150, 9.5 Hz, 4F, **F_{eq}**).

**HR-FAB-MS** m/z (calc.) = 425.1574 [M-Ph^{·+}]; m/z (found) = 425.1576 [M-Ph^{·+}] (C₂₀H₂₆O₂F₅S); m/z (calc.) = 405.1312 [M-OHex^{·+}]; m/z (found) = 405.1312 [M-Hex^{·+}] (C₂₀H₂₂O₁F₅S); m/z (calc.) = 375.2324 [M-SF₅^{·+}]; m/z (found) = 375.2323 [M-SF₅^{·+}] (C₂₆H₃₁O₂).

### Beispiel 23: 7-(Hex-5-in-1-yloxy)-5-((pentafluor-λ⁶-sulfanyl)methylen)-7-phenyloctan-1-ol

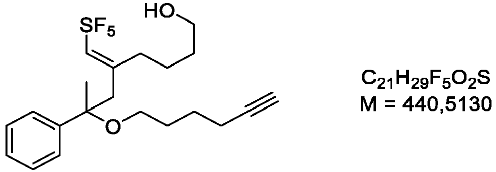

Die Verbindung wurde nach der allgemeinen Reaktionsvorschrift A hergestellt. In einem Glasrohr mit Young-Hahn wurden 26,0 µl (23,6 mg, 0,200 mmol, 1,00 eq.) α-Methylstyrol sowie 441 µl (393 mg, 2,00 mmol, 20,0 eq.) 5-Hexin-1-ol eingesetzt. Die Verbindung wurde durch ein Säulenchromatographie-System (C18, 65% MeCN in Wasser bis 71% MeCN in Wasser) vorgereinigt und die Produktfraktion durch Säulenchromatographie (Silica, DCM) gereinigt. Die Ausbeute des Produkts wurde durch die Analyse des Rohreaktionsgemischs mittels ¹⁹F-NMR-Spektroskopie (31%) bestimmt. Das Produkt wurde als farbloses Öl gewonnen.

**¹H NMR** (500 MHz, Chloroform-*d*) δ 7.37 - 7.31 (m, 4H), 7.30 - 7.25 (m, 1H), 6.05 (p, *J* = 9.4 Hz, 1H, C=**CHSF₅**), 3.60 (t, *J* = 6.2 Hz, 1H,**CH₂**OH), 3.31 (dt, *J* = 9.0, 6.0 Hz, 1H, O**_{CH2-dia}**), 3.11 (dt, *J* = 8.9, 5.9 Hz, 1H, O**_{CH2-dia}**), 2.49 (d, *J* = 13.7 Hz, 1H, Cq**CH_{2,dia}**Cq), 2.39 (d, *J* = 13.7 Hz, 1H, Cq**CH_{2,dia}**Cq), 2.29 - 2.19 (m, 4H, 2 × **CH₂**), 1.96 (t, *J* = 2.7 Hz, 1H, C≡**C-H**), 1.71 - 1.62 (m, 4H, 2 × **CH₂**), 1.61 (s, 3H, **CH₃**), 1.54 - 1.34 (m, 4H, 2 × **CH₂**).

**¹³C NMR** (126 MHz, Chloroform-*d*) δ 145.62 (p, *J* = 4.8 Hz, **C_{q}**=CHSF₅), 144.18 (**C**_{q,Ar}), 138.73 (p, *J* = 17.2 Hz, C_{q}=**CH**SF₅), 128.47 (**CH**_{Ar}), 127.46 (**CH**_{Ar}), 126.14 (**CH**_{Ar}), 84.43 (**C≡**CH), 78.85 (C_{q,bridge}), 68.58 (C≡**CH**), 62.52 (O**CH₂**), 61.91 (O**CH₂**), 49.32 (C_{q,asymm}**CH₂**C_{q}), 32.78 (OCH₂**CH₂**), 32.57 (SF₅CH=C_{q}**CH₂**CH₂), 29.34 (**CH**_{**2,**int}), 25.43 (**CH**_{**2,**int}), 24.02 (**CH**_{**2,**int}), 22.52 (**CH**_{**2,**int}), 18.37 (**CH₂**C≡CH).

**¹⁹F NMR** (471 MHz, Chloroform-*d*) δ 87.02 - 85.38 (m), 65.64 (dd, *J* = 149.9, 9.5 Hz). (40er series, 07.08.19).

**HR-EI-MS** m/z (calc.) = 425.1574 [M-CH₃^{·+}]; m/z (found) = 425.1575 [M-CH₃^{·+}].

### Darstellung von 4-((Pentafluor-λ⁶-sulfanyl)-methylen)-2-phenyl-oxacycloalkanen:

### Beispiel 24: 4-((Pentafluor-λ⁶-sulfanyl)-methylen)-2,2-diphenyloxepan

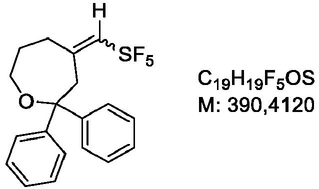

In einem Glasrohr mit Young-Hahn wurden 35,3 µl (36,1 mg, 0,20 mmol, 1,00 eq.) 1,1-Diphenylethylen sowie 186 µl (168 mg, 2,00 mmol, 10,0 eq.) 4-Pentin-1-ol in 1 ml Sureseal^{®} Acetonitril (filtriert) gelöst. Anschließend wurden 5,5 mg (0,2 mmol, 10 mol%) N-Phenylphenothiazin zugegeben sowie 20 µl Triethylboran. Die Reaktionsmischung wurde durch drei *freeze-pump-thaw-Zyklen* entgast und die Atmosphäre wurde gegen Schwefelhexafluorid getauscht. Die Reaktionsmischung wurde für 22 h bei 20°C unter Belichtung bei 368 nm gerührt. Anschließend wurde das Produkt durch ¹⁹F-NMR-Spektroskopie quantifiziert und durch Säulenchromatographie aufgereinigt (Silica, 5% DCM in Hexan). Das Produkt wurde als hochviskoses Öl (28%) erhalten.

**¹H NMR** (500 MHz, Chloroform-*d*) δ 7.40 - 7.34 (m, 4H, **CH**_{Ar}), 7.30 (td, *J* = 6.9, 6.3, 2.0 Hz, 4H, **CH_{Ar}**), 7.25 - 7.20 (m, 2H, **CH_{Ar}**), 5.47 - 5.13 (p, J = 9.2 Hz, C=**CH**SF₅), 3.79 - 3.69 (m, 2H, O**-CH₂**), 3.16 (s, 2H, C_{q}-**CH₂**-C_{q}), 2.67 - 2.58 (m, 2H, C_{q,DB}**CH₂**CH₂), 1.88 (ddd, *J* = 11.5, 8.7, 5.8 Hz, 2H, CH₂**CH₂**CH₂).

**¹³C NMR** (126 MHz, Chloroform-*d*) δ 145.64 (**C**_{q,Ar})" 142.63 (p, *J* = 5.0 Hz, **C**_{q}=CHSF₅), 137.79 (p, *J* = 18.2 Hz, **C**_{q}=**CH**SF₅), 128.23 (**CH**_{Ar}), 127.27 (**CH**_{Ar}), 126.78 (**CH**_{Ar}), 82.04 (**C**_{q,Brücke}), 63.59 (O-**CH₂**) , 48.84 (C_{q}-**CH₂**-C_{q}), 32.39 (C_{q,DB}**CH₂**CH₂) , 28.26 (CH₂**CH₂**CH₂).

**¹⁹F NMR** (471 MHz, Chloroform-*d*) δ 85.35 (p, *J* = 149.0, 1F, **Fₐₓ**), 65.36 (dd, *J* = 150.0, 9.5 Hz, 4F, **F_{eq}**).

**HR-EI-MS** m/z (calc.) = 390.1077 [M˙⁺]; m/z (found) = 390.1077 [M˙⁺] (C₁₉H₁₉F₅OS). Wurde aus der Isomerenmischung bestimmt.

### Beispiel 25: 4-((Pentafluor-λ⁶-sulfanyl)-methylen)-2,2-diphenyltetrahydro-2H-pyran

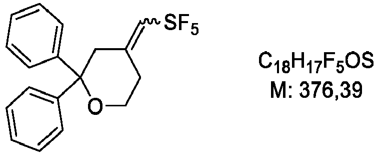

In einem Glasrohr mit Young-Hahn wurden 35.3 µl (36,1 mg, 0,20 mmol, 1,00 eq.) 1,1-Diphenylethylen sowie 151 µl (140 mg, 2,00 mmol, 10,0 eq.) 3-Butin-1-ol in 1 mL filtriertem Sureseal^{®} Acetonitril gelöst. Anschließend wurden 5,5 mg (0,2 mmol, 10 mol%) Phenylphenothiazin zugegeben sowie 20 µl Triethylboran. Die Reaktionsmischung wurde durch drei *freeze-pump-thaw-Zyklen* entgast und die Atmosphäre wurde gegen Schwefelhexafluorid getauscht. Die Reaktionsmischung wurde für 22 h bei 20°C unter Belichtung bei 368 nm gerührt. Anschließend wurde das Produkt durch ¹⁹F-NMR-Spektroskopie quantifiziert und durch Säulenchromatographie aufgereinigt (Silica, 10% DCM in Hexan). Das Produkt wurde als hochviskoses Öl (24%) erhalten.

**¹H NMR** (500 MHz, CDCl3) δ = 7.36 - 7.19 (m, 10H, **CH_{Ar}**), 6.39 (p, J=8.6, 7.8, 1H, C=**CH**SF₅), 3.77 (t, J=5.7, 2H, O-**CH₂**), 2.97 (s,2H, C_{q}-**CH₂**-C_{q}), 2.68 (t, J=5.7, 2H, C_{q,DB}**CH₂**CH₂).

**¹³C NMR** (126 MHz, CDCl3) δ = 144.54 (**C**_{q,Ar}), 142.42 (p, J = 5.1 Hz, **C**_{q}=CHSF₅), 135.76 (p, *J* = 18.4, C_{q}=**CH**SF₅), 128.54 (**CH**_{Ar}), 127.40 (**CH**_{Ar}), 126.56 (**CH**_{Ar}), 81.20 (**C**_{q,Brücke}), 61.48 (O-**CH₂**), 44.39 (C_{q}-**CH₂**-C_{q}), 30.75 (CH₂-**CH₂**-C_{q}).

**¹⁹F-NMR** (471 MHz, CDCl3) δ = 85.36 (p, J = 149.9 Hz, 1 F, **Fax**), 66.58 (d, J = 150.1 Hz, 8.2 Hz, 4 F, **F_{eq}**).

**HR-EI-MS** m/z (calc.) = 376.0920 [M'+]; m/z (found) = 376.0921 [M'+] (C₁₈H₁₇F₅OS). Wurde aus der Isomerenmischung bestimmt.

### Beispiel 26: 2-Methyl-4-((pentafluor-λ⁶-sulfanyl)-methylen)-2-phenyloxepan

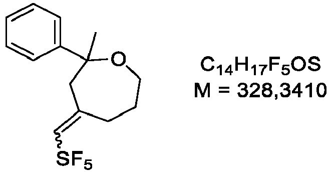

In einem Glasrohr mit Young-Hahn wurden 26.0 µl (11,8 mg, 0,20 mmol, 1,00 eq.) α-Methylstyrol sowie 186 µl (2,00 mmol, 10,0 eq.) 4-Pentin-1-ol in 1 ml Sureseal^{®} Acetonitril (filtriert) gelöst. Anschließend wurden 5,5 mg (0,02 mmol, 5 mol%) Phenylphenothiazin zugegeben und 20 µl (0,20 mmol, 10 mol%) Triethylboran (1M in Hexan) zur Reaktionsmischung gegeben. Die Reaktionsmischung wurde durch drei *freeze-pump-thaw*-Zyklen entgast und die Atmosphäre wurde gegen Schwefelhexafluorid getauscht. Die Reaktionsmischung wurde für 22 h bei 20°C unter Belichtung bei 368 nm gerührt. Anschließend wurde das Produkt durch ¹⁹F-NMR-Spektroskopie quantifiziert und durch Säulenchromatographie (SiO₂, Hexan (10% DCM in Hexan) gereinigt. Das Produkt wurde als farbloses viskoses Öl (17 %) erhalten.

**¹H NMR** (500 MHz, CDCl3) δ = 7.35 - 7.10 (m, 5H, **CH_{Ar}**) 6.03 (p, J=9.1, 1H, C=**CH**SF₅), 3.88 (dt, J=13.1, 4.5, 1H, O-**CH_{2,dia}**), 3.59 (ddd, J=12.4, 8.1, 3.4, 1H, O-**CH_{2,dia}**), 2.90 (m, 1H, C_{q}-**CH_{2,dia}**-CH₂), 2.80 (s, 1H, C_{q}-**CH₂**-C_{q}), 2,30 (dt, J = 7.28, 14.70, 1H, C_{q}-**CH_{2,dia}**-CH₂), 1.80 (d, J=5.1, 2H, CH₂-**CH_{2,dia}**-CH₂), 1.46 (s, 3H, **CH₃**).

**¹³C NMR** (126 MHz, CDCl3) δ 146.48 (**C**_{q,Ar}), 143.89 (J = 5.8 Hz, **C**_{q}=CHSF₅), 137.28 (J=17.2 Hz, C_{q}=**CH**SF₅), 128.02 (**CH**_{Ar}),, 126.55 (**CH**_{Ar}),, 124.68 (**CH**_{Ar}), 63.33 (O-**CH₂**), 47.76 (C_{q}-**CH₂-**C_{q}),, 31.21 (CH₂-**CH₂**-C_{q})., 29.66 (**CH₃**), 27.90 (CH₂-**CH₂-C_{q}).**

**¹⁹F NMR** (126 MHz, CDCl3) δ = 86.36 - 85.23 (p, J = 149.8 Hz,1 F, **Fₐₓ**), 65.94 (dd, J=150.2, 11.6, 4 F, **F_{eq}**).

**HR-EI-MS** m/z (calc.) = 328.0920 [M^{•+}]; m/z (found) = 328.0922 [M ^{•+}] (C₁₄H₁₇F₅OS). Aus der Isomerenmischung bestimmt.

Allgemeine Reaktionsvorschrift B zur Herstellung weiterer **4-((Pentafluor-λ⁶**-**sulfanyl)-methylen)-2-phenyl-oxacycloalkanen:**
In einem Glasrohr mit Young-Hahn wurden 0,20 mmol (1,00 eq.) des Alkens zu 1 ml Acetonitril (Surseal-Grad über Molekularsieben gelagert, gefiltert) zugegeben. Dann wurden 2,00 mmol (10,0 eq.) des Alkohols sowie 5,50 mg (0,02 mmol, 10 mol%) des Photoredox-Katalysators hinzugefügt. Schließlich wurden 20 µl (0,02 mmol, 10 mol%) BEt₃ (1,0 M in Hexan) dem Reaktionsgemisch zugegeben. Das Reaktionsgemisch wurde durch drei *freeze-pump-thaw-*Zyklen entgast und mit Schwefelhexafluorid (2,8 bar, 15 Äquivalente) nachgefüllt. Das Reaktionsgemisch wurde bei Raumtemperatur unter Bestrahlung bei 368 nm durch eine Hochleistungs-LED bei 20 °C für 22 h gerührt. Die Ausbeute wurde durch ¹⁹F-NMR-Spektroskopie mit α,α,α-Trifluorotoluol als internem Standard bestimmt. Die Verbindungen wurden mittels Säulenchromatographie mit Siliziumdioxid als stationäre Phase und Dichlormethan in Hexan bzw. in Cyclohexan als Elutionsmittel gereinigt.

### Beispiel 27: 4-((Pentafluor-λ⁶-sulfanyl)methylen)-2,2-diphenyltetrahydrofuran

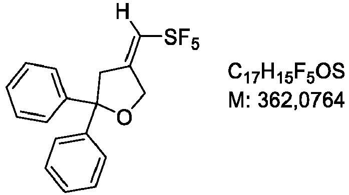

Die Verbindung wurde nach der allgemeinen Reaktionsvorschrift B hergestellt. In einem Glasrohr mit Young-Hahn wurden 35,3 µl (0,200 mmol, 1,00 eq.) Diphenylethylen sowie 237 µl (224 mg, 4,00 mmol, 20,0 eq.) Propargylalkohol eingesetzt. Die Verbindung wurde durch ein Säulenchromatographiesystem (C18, 60% MeCN in Wasser bis 78% MeCN in Wasser) vorgereinigt und die Produktfraktion durch Säulenchromatographie (Silica, 5% DCM in Hexan bis 10% DCM in Hexan) gereinigt. Die Ausbeute des Produkts wurde durch die Analyse des Rohreaktionsgemischs mittels ¹⁹F-NMR-Spektroskopie bestimmt. Das Produkt wurde als farbloser Feststoff erhalten.

**¹H NMR** (500 MHz, Chloroform-*d*) δ 7.42 - 7.37 (m, 4H, **CH_{Ar}**), 7.32 (ddd, *J* = 7.8, 6.8, 1.2 Hz, 4H, **CH_{Ar}**), 7.27 - 7.23 (m, 2H, **CH_{Ar}**), 6.48 (tp, *J* = 8.9, 2.4 Hz, 1H, C=**CH**SF₅), 4.72 (p, *J =* 2.4 Hz, 1H, O-**CH₂**), 3.41 (dp, *J* = 4.1, 2.1 Hz, 1H, C_{q}-**CH₂**-Cq).

**¹³C NMR** (126 MHz, Chloroform-*d*) δ 148.83 (**C_{q}**=CHSF₅), 143.56 (**C**_{q,Ar}), 131.13 (p, *J* = 21.2 Hz, C_{q}=**CH**SF₅), 128.66 (**CH**_{Ar}), 127.68 (**CH**_{Ar}), 125.89 (**CH**_{Ar}), 87.82 (**C**_{q,Brücke}), 68.90 (O-**CH₂**), 45.59 (C_{q}-**CH₂**-C_{q}).

**¹⁹F NMR** (471 MHz, Chloroform-*d*) δ 83.15 (p, *J* = 149.3, 1F, **Fₐₓ**), 62.88 (dd, *J* = 149.3, 9.0 Hz, 4 F, **F_{eq}**).

**HR-EI-MS** m/z (calc.) = 362.0763 [M'+]; m/z (found) = 362.0764 [M^{•+}] (C₁₇H₁₅F₅OS).

### Beispiel 28: 2-Methyl-4-((pentafluor-λ⁶-sulfanyl)methylen)-2-phenyltetrahydro-2H-pyran

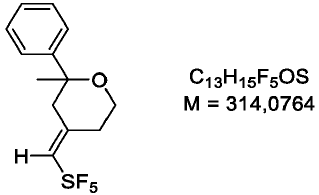

Die Verbindung wurde nach der allgemeinen Reaktionsvorschrift B hergestellt. In einem Glasrohr mit Young-Hahn wurden 26,0 µl (23,6 mg, 0,200 mmol, 1,00 eq.) α-Methylstyrol sowie 303 µl (280 mg, 4,00 mmol, 20,0 eq.) 3-Butin-1-ol eingesetzt. Die Verbindung wurde durch ein Säulenchromatographiesystem (C18, 65% MeCN in Wasser bis 73% MeCN in Wasser) vorgereinigt und die Produktfraktion durch Säulenchromatographie (Silica, Cyclohexan bis 10% DCM in Cyclohexan) gereinigt. Die Ausbeute des Produkts wurde durch die Analyse des Rohreaktionsgemischs mittels 19F-NMR-Spektroskopie bestimmt. Das Produkt wurde als farbloses Öl gewonnen.

**¹H NMR** (500 MHz, Chloroform-*d*) δ 7.41 - 7.34 (m, 4H), 7.31 - 7.23 (m, 1H), 6.29 (p, *J* = 8.9 Hz, 1H, C=**CH**SF₅), 3.87 (ddd, *J* = 11.7, 5.9, 4.9 Hz, 1H, O-**CH_{2,dia}**-CH₂), 3.70 (ddd, *J* = 11.9, 7.6, 4.6 Hz, 1H, O-**CH_{2,dia}**-CH₂), 2.79 (d, *J* = 13.7 Hz, 1H, Cq - **CH_{2,dia}**), 2.70 - 2.57 (m, 2H, O-CH_{2,dia}-**CH₂**), 2.51 (dq, *J* = 13.8, 1.6 Hz, 1H, Cq - **CH_{2,dia}**), 1.48 (s, 3H, **CH₃**).

**¹³C NMR** (126 MHz, Chloroform-d) δ 144.68 (**C**_{q,Ar}), 142.74 (p, *J* = 5.3 Hz, , **C**_{q}=CHSF₅), 135.16 (p, *J* = 18.1 Hz, C_{q}=**CH**SF₅), 128.68 (**CH**_{Ar}), 127.33 (**CH**_{Ar}), 125.35 (**CH**_{Ar}), 77.56, 61.50 (O-**CH_{2,dia}**), 44.55 (C_{q}-**CH_{2,dia}**), 30.98 (p, *J* = 3.6 Hz, C_{q}-CH_{2,dia}-**CH₂**), 29.31 (**CH₃**).

**¹⁹F NMR** (126 MHz, CDCl3) δ = 85.13 (p, J = 150 Hz, 1F, **Fₐₓ**), 66.12 (dd, J=150, 8.7, 4 F, **F_{eq}**).

**HR-EI-MS** m/z (calc.) = 314.0764 [M'+]; m/z (found) = 314.0765 [M ^{•+}] (C₁₃H₁₅F₅OS).

### Beispiel 29: 2-Methyl-4-((pentafluor-X6-sulfanyl)methylen)-2-phenyltetrahydro-furan

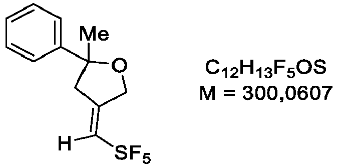

Die Verbindung wurde nach der allgemeinen Reaktionsvorschrift B hergestellt. In einem Glasrohr mit Young-Hahn wurden 26,0 µl (23,6 mg, 0,200 mmol, 1,00 eq.) α-Methylstyrol sowie 237 µl (224 mg, 4,00 mmol, 20,0 eq.) Propargylalkohol eingesetzt. Die Verbindung wurde durch ein Säulenchromatographiesystem (C18, 65% MeCN in Wasser bis 72% MeCN in Wasser) vorgereinigt und die Produktfraktion durch Säulenchromatographie (Silica, Cyclohexan bis 10% DCM in Cyclohexan) gereinigt. Die Ausbeute des Produkts wurde durch die Analyse des Rohreaktionsgemischs mittels ¹⁹F-NMR-Spektroskopie bestimmt. Das Produkt wurde als farbloses Öl gewonnen.

**¹H NMR** (500 MHz, Chloroform-*d*) δ 7.41 - 7.33 (m, 4H, **CH**_{Ar}), 7.30 - 7.27 (m, 1H, **CH**_{Ar}), 6.41 (ddddp, *J* = 9.0, 2.3 Hz, 2.3 Hz, 2.3 Hz, 2.3 Hz, 1H, C_{q}=**CH**SF₅), 4.81 (dp, *J* = 16.8, 2.0 Hz, 1H, **CH**_{**2**,dia}), 4.62 (dp, *J* = 18.3, 2.7, 2.2 Hz, 1H, **CH_{2,dia}**), 3.16 - 3.05 (m, 1H, **CH**_{**2**,dia}), 2.90 (dp, *J* = 16.2, 2.5 Hz, 1H, **CH**_{**2**,dia}), 1.58 (s, 3H, **CH₃**).

**¹³C NMR** (126 MHz, Chloroform-d) δ 149.35 (p, *J* = 5.5 Hz, **C**_{q}=CHSF₅), 144.64 (**C**_{q,Ar}), 131.24 (p, *J* = 20.9 Hz, C_{q}=**CH**SF₅), 128.69 (**CH**_{Ar}), 127.42 (**CH**_{Ar}), 124.76 (**CH**_{Ar}), 84.52 (**C**_{**q**,bridge}), 68.96 (p, *J* = 3.8 Hz, **CH₂**), 46.05 (**CH₂**), 28.83 (**CH₃**).

**¹⁹F NMR** (471 MHz, Chloroform-*d*) δ 83.80 - 82.49 (m, 1 F, **Fₐₓ**), 62.78 (dd, *J* = 149.2, 9.2 Hz, 4 F, **F_{eq}**).

**HR-EI-MS** m/z (calc.) = 300.0607 [M'+]; m/z (found) =300.0609 [M ^{•+}] (C₁₂H₁₃OF₅S); m/z (calc.) = 285.0371 [M-CH₃^{•+}]; m/z (found) = 285.0373 [M-CH₃^{•+}] (C₁₁H₁₀OF₅S). **Eliminierung** von Pentafluoro-(2-alkoxy-2-phenylethyl)-λ⁶-sulfanen zu Pentafluoro-(2-phenyl-ethylen)-λ⁶-sulfanen

### Beispiel 30: (2,2-Diphenylvinyl)-pentafluoro-λ⁶-sulfan

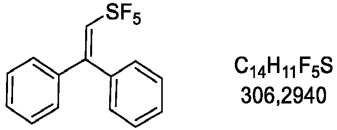

In einem headspace vial wurde das Pentafluoro-(2-methoxy-2,2-diphenylethyl)-λ⁶-sulfan (9,3 µmol) in 500 µl CDCl₃ unter Atmosphäre gelöst und anschließend BF₃·Et₂O (50,0 µL, 21,2 eq.) zugegeben. Die Reaktionsmischung wurde drei Stunden bei Raumtemperatur gerührt. Die Reaktion wurde durch ¹⁹F-NMR-Spektroskopie verfolgt. Die Ausbeute ist > 90%.

**¹H NMR** (300 MHz, Chloroform-d) δ 7.43 - 7.28 (m, 6H, **CH**_{Ar}), 7.25 - 7.19 (m, 4H, **CH**Ar), 6.87 (p, *J* = 8.4 Hz, 1H, C=C**H**-SF₅).

**¹³C{¹H} NMR** (101 MHz, Chloroform-d) δ 147.64 (q, *J* = 5.7 Hz, **C**_{q,Brücke}), 139.03 (**C**_{q,Ar}), 137.55 (q, *J =* 18.3 Hz, **CH₂**-SF₅), 137.32 (**CH**_{Ar}), 129.80 (CH_{Ar}) 129.80 (**CH**_{Ar}), 128.58 (q, *J =* 1.8 Hz, **C**_{q,Ar}), 128.31 (**CH**_{Ar}), 128.14 (**CH**_{Ar}), 128.06 (**CH**_{Ar}).

**¹⁹F NMR** (471 MHz, Chloroform-d) δ 84.80 - 83.18 (m, 1F, **Fₐₓ**), 68.59 (dd, *J* = 152.4, 8.5 Hz, 4F, **F_{eq}**).

**HR-EI-MS** m/z (calc.) = 306.0500 [M^{•+}]; m/z (found) = 306.0502 [M^{•+}] (C₁₄H₁₁F₅S).

### Beispiel 31: Pentafluor-(2-phenylallyl)-λ⁶-sulfan

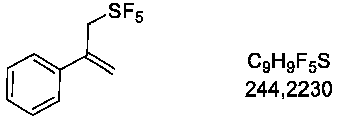

In einem Borosilikat-NMR-Röhrchen wurden 5,2 mg (19 µmol, 1,00 eq.) von 8 in 400 µl CDCl₃ suspendiert. Dann wurden 23,8 µl (188 µmol, 10,0 eq.) BFs-OEt zugegeben. Das Rohr wurde mit Parafilm versiegelt und das Reaktionsgemisch stark geschüttelt. Anschließend wurde der Reaktionsfortschritt von ¹⁹F-NMR über einen Zeitraum von 27 min überwacht, bis das Ausgangsmaterial vollständig in das Produkt umgewandelt wurde. Die Ausbeute des Produktes wurde mittels ¹⁹F-NMR-Spektroskopie (> 98%) bestimmt. Die Identität des Produkts wurde mittels ¹H-NMR, ¹⁹F-NMR sowie HSQC_{ed}- und ¹H-¹³C-HMBC-Spektroskopie bestätigt.

**¹H NMR** (400 MHz, Chloroform-d) δ 7.46 - 7.32 (m, 5H, **CH**_{Ar}), 5.77 (s, 1H, C=CHₐ**H_{b}**), 5.56 (s, 1H, C=C**HₐH**b), 4.71 (p, *J =* 7.6 Hz, 2H, Cq-C**H**₂-SF₅).

**¹³C{¹H} NMR** (101 MHz, Chloroform-d) δ 139.2 (**C_{q}**), 138.6 (**C_{q}**), 128.8 (**CH**_{Ar}), 128.5 (**CH**_{Ar}), 126.2 (**CH**_{Ar}), 123.9 (**CH**_{Ar}), 75.4 (p, *J =* 13.4 Hz, **CH₂**SF₅).

**¹⁹F NMR** (471 MHz, Chloroform-d) δ 82.77 - 81.47 (m, 1F, **Fₐₓ**), 64.65 (dt, *J* = 145.5, 7.7 Hz, 4F, **F_{eq}**).

**HR-EI-MS** m/z (calc.) = 244.0 [M'+]; m/z (found) = 243.9 [M^{•+}] (C₉H₁₉F₅S).

Die vorliegende Erfindung stellt eine effiziente, nachhaltige und umweltfreundliche photoredoxkatalysierte Pentafluorsulfanylierungsmethode bereit. Die einzigartige Kombination aus photochemischem Abbau des starken Treibhausgases Schwefelhexafluorid unter Darstellung synthetisch wertvoller Verbindungen als pentafluorsulfanylierte Bausteine leistet einen wesentlichen Beitrag zur nachhaltigen Chemie. Die Zugänglichkeit derartiger Verbindungen war bislang durch die Verfügbarkeit und Toxizität der benötigten Reagenzien limitiert.

Das erfindungsgemäße Verfahren ermöglicht die Addition einer Pentafluorsulfanyl- und einer Alkoxy-Gruppe an eine ungesättigte Kohlenstoff-Kohlenstoff-Bindung. Ohne durch eine bestimmte Theorie eingeschränkt zu sein, wird angenommen, dass es sich bei der Reaktion um einen konsekutiven Zweielektronenabsorptionsprozess handelt. In einem ersten Anregungsprozess wird der Photoredoxkatalysator (besonders bevorzugt N-Phenylphenothiazine) angeregt und ein erster Elektronentransfer auf SF₆ initiiert. Das entstehende Radikalkation des Katalysators wird erneut angeregt und oxidiert final das Substrat (Edukt) unter Rückbildung des Katalysators. Dieser Mechanismus entspricht dem Prinzip der Photoredoxkatalyse. Gemäß der vorliegenden Erfindung findet zum einen eine selektive Aktivierung durch Reduktion des Schwefelhexafluorids mithilfe von Licht sowie ein atomökonomischer Abbau von Schwefelhexafluorid unter Bildung des pentafluorsulfanylierten Produkts statt. Somit werden pentafluorsulfanylierte Produkte, zu denen wertvolle und pharmakologisch potentiell wichtige Verbindungen zählen, effizient, nachhaltig und umweltschonend erhalten. Das erfindungsgemäße Verfahren erzielt für bestimmte Substrate durch die Anregung des Photoredoxkatalysators durch Licht die Addition einer SF₅-Gruppe und einer Alkoxy-Gruppe an eine ungesättigte Kohlenstoff-Kohlenstoff-Bindung mit hohen Ausbeuten. Erfindungsgemäß wird durch den Zusatz von Triethylboran ein selektiver Reaktionsverlauf zu den alpha-alkoxypentafluorsulfanylierten Produkten erhalten. Weiterhin werden mit dem erfindungsgemäßen Verfahren ((Pentafluorsulfanyl)-methylen)-oxacycloalkane durch Addition eines terminalen Alkinylalkohols an eine ungesättigte Kohlenstoff-Kohlenstoff-Bindung und Addition eines Pentafluorsulfanylradikals (·SF₅) an die entstehende exocyclische Methylengruppe, sowie Pentafluorsulfanylalkene durch Eliminierung von vicinalen Pentafluorsulfanylethern erhalten. Zudem ist es möglich, durch das erfindungsgemäße Verfahren das umweltschädliche Treibhausgas SF₆ abzubauen.

## Patentansprüche

1. Verfahren zur photoredoxkatalytischen alpha-Alkoxypentafluorsulfanylierung eines Edukts mit ungesättigter Kohlenstoff-Kohlenstoff-Bindung, umfassend die Schritte
(A) Bereitstellen eines Reaktionsgemisches, welches das Edukt, einen Alkohol ROH, einen Photoredoxkatalysator sowie Schwefelhexafluorid, umfasst; und
(B) Bestrahlen des Reaktionsgemisches mit elektromagnetischer Strahlung, welche UV-Licht und/oder Licht aus dem sichtbaren Wellenlängenbereich ist.
wobei R ausgewählt ist aus einer gesättigten oder ungesättigten, substituierten oder unsubstituierten, unverzweigten oder verzweigten Kohlenwasserstoffgruppe.

2. Verfahren nach Anspruch 1, wobei
der Photoredoxkatalysator ein Photoredoxkatalysator mit einer N-Phenylphenothiazin-Grundstruktur ist.

3. Verfahren nach Anspruch 1 oder 2, wobei
der Photoredoxkatalysator eine Verbindung der folgenden Formel (1) umfasst 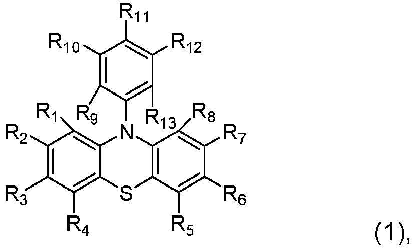 wobei die Reste R₁ bis R₁₃ unabhängig voneinander ein Wasserstoffatom, ein Deuteriumatom, ein Halogenatom, eine Cyanogruppe, eine Nitrogruppe, eine Trifluormethylgruppe, ein Fluoratom, eine substituierte oder unsubstituierte Kohlenwasserstoffgruppe, welche gegebenenfalls ein oder mehrere Heteroatome enthält, oder einen substituierten oder unsubstituierten aromatischen Rest darstellen.

4. Verfahren nach einem der Ansprüche 1 bis 3, wobei
der Photoredoxkatalysator N-Phenylphenothiazin ist.

5. Verfahren nach einem der Ansprüche 1 bis 4, wobei
die ungesättigte Kohlenstoff-Kohlenstoff-Bindung eine Doppelbindung ist.

6. Verfahren nach einem der Ansprüche 1 bis 5, wobei das Edukt eine Verbindung der folgenden Formel (I) umfasst 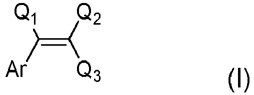 wobei
die Reste Q₁ bis Q₃ unabhängig voneinander ein Wasserstoffatom, ein Deuteriumatom, eine Cyanogruppe, eine Trifluormethylgruppe, eine substituierte oder unsubstituierte Kohlenwasserstoffgruppe, welche gegebenenfalls ein oder mehrere Heteroatome enthält, oder einen substituierten oder unsubstituierten aromatischen Rest darstellen und Ar ein aromatischer Rest ist.

7. Verfahren nach einem der Ansprüche 1 bis 6, wobei
das Edukt eine Verbindung mit einer Styrol-Grundstruktur ist.

8. Verfahren nach einem der Ansprüche 1 bis 7, wobei
die elektromagnetische Strahlung Licht aus dem sichtbaren Wellenlängenbereich mit einer Wellenlänge von 400 bis 500 nm und/oder UV-A Licht ist.

9. Verfahren nach einem der Ansprüche 1 bis 8, wobei
das Reaktionsgemisch weiterhin eine Lewis-Säure enthält.

10. Verfahren nach Anspruch 9, wobei
die dem Reaktionsgemisch zugesetzte Lewis-Säure Triethylboran ist.

11. Verfahren nach einem der Ansprüche 1 bis 10, wobei,
wenn die Gruppe R des Alkohols ROH eine Alkinylalkylgruppe HCC(CH₂)ₙ- mit n > 1 ist, in Schritt (B) neben dem vicinalen Pentafluorsulfanylether ein 4-((Pentafluor-λ⁶-sulfanyl)-methylen)-oxacycloalkan gebildet wird.

12. Verfahren zur Darstellung von Pentafluor-(ethylen)-λ⁶-sulfanen ausgehend von den aus dem Verfahren gemäß einem der Ansprüche 1-10 erhaltenen Pentafluor-(2-alkoxyethyl)-λ⁶-sulfanen, umfassend die Schritte
(C) Bereitstellen eines Reaktionsgemisches, welches als Edukt einen vicinalen Pentafluorsulfanylether, eine starke Lewis-Säure und ein Lösungsmittel umfasst;
und
(D) Rühren des Reaktionsgemisches; und
(E) Quenchen des Reaktionsgemisches mit einer wässrigen Lösung und Extraktion des Produktes mit einem organischen Lösungsmittel.

13. Verfahren nach Anspruch 12, wobei
der vicinale Pentafluorsulfanylether eine Verbindung der folgenden Formel (III) umfasst 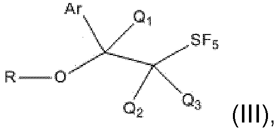 wobei
die Reste Q₁ bis Q₃ unabhängig voneinander ein Wasserstoffatom, ein Deuteriumatom, eine Cyanogruppe, eine Trifluormethylgruppe, eine substituierte oder unsubstituierte Kohlenwasserstoffgruppe, welche gegebenenfalls ein oder mehrere Heteroatome enthält, oder einen substituierten oder unsubstituierten aromatischen Rest darstellen, Ar ein aromatischer Rest ist und R eine gesättigte oder ungesättigte, substituierte oder unsubstituierte, unverzweigte oder verzweigte Kohlenwasserstoffgruppe ist.

14. Verfahren nach Anspruch 13, wobei
der vicinale Pentafluorsulfanylether eine Verbindung mit einer Styrol-Grundstruktur ist.

15. Verfahren nach einem der Ansprüche 12 bis 14, wobei die starke Lewis-Säure im Reaktionsgemisch BF₃·Et₂O darstellt.

## Claims

1. Method for the photoredox catalytic alpha-alcoxypentafloursulphonation of an educt with unsaturated carbon-carbon bonding, comprising the steps:
(A) providing a reaction mixture comprising the educt, an ROH alcohol, a photoredox catalyst, and sulphahexafluoride;
and
(B) irradiating the reaction mixture with electromagnetic radiation, which is UV light and/or light from the visible wavelength range,
wherein R is selected from a saturated or unsaturated, substituted or unsubstituted, branched or unbranched hydrocarbon group.

2. Method according to claim 1, wherein the photoredox catalyst is a photoredox catalyst with an N-phenyl phenothiazine basic structure.

3. Method according to claim 1 or 2, wherein the photoredox catalyst comprises a compound with the following formula (1) 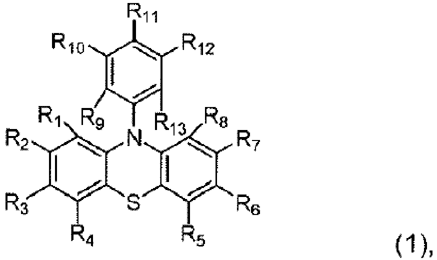 wherein the remainder R₁ to R₁₃ independently represent a hydrogen atom, a deuterium atom, a halogen atom, a cyano group, a nitro group, a trifluormethyl group, a fluorine atom, a substituted or unsubstituted hydrocarbon group, which optionally contain one or more heteroatoms, or represent a substituted or unsubstituted aromatic residue.

4. Method according to any one of claims 1 to 3, wherein the photoredox catalyst is N-phenyl phenothiazine.

5. Method according to any one of claims 1 to 4, wherein the unsaturated carbon-carbon bond is a double bond.

6. Method according to any one of claims 1 to 5, wherein the educt comprises a bond with the following formula (I) 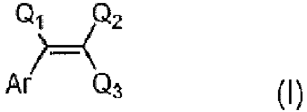 wherein the remainder Q₁ to Q₃ independently represent a hydrogen atom, a deuterium atom, a cyano group, a trifluormethyl group, a substituted or unsubstituted hydrocarbon group, which optionally contain one or more heteroatoms, or represent a substituted or unsubstituted aromatic residue, and Ar is an aromatic residue.

7. Method according to any one of claims 1 to 6, wherein the educt is a compound with a styrene basic structure.

8. Method according to any one of claims 1 to 7, wherein the electromagnetic radiation is light from the visible wavelength range, with a wavelength of 400 to 500 nm and/or is UV-A light.

9. Method according to any one of claims 1 to 8, wherein the reaction mixture further contains a Lewis acid.

10. Method according to claim 9, wherein the Lewis acid which is added to the reaction mixture is triethylborane.

11. Method according to any one of claims 1 to 10, wherein
if the group R of the ROH alcohol is an alkinyl alkyl group HCC(CH₂)ₙ-, where n > 1, and in step (B), then, in addition to the vicinal pentafluorsulphane ether, a 4-((pentaflour-λ⁶-sulphanyl)- (methylene)-oxacycloalkane is formed.

12. Method for representing pentaflour-(ethylene)-λ⁶-sulphane, deriving from the pentaflour-(2-alcoxethyl)-λ⁶-sulphane obtained from the method according to any one of claims 1-10, comprising the steps
(C) providing a reaction mixture, which comprises as an educt a vicinal pentafluorsulphane ether, a strong Lewis acid, and a solvent;
and
(D) stirring the reaction mixture; and
(E) quenching the reaction mixture with an aqueous solution, and extraction of the product with an organic solvent.

13. Method according to claim 12, wherein the vicinal pentafluorsulphane ether comprises a bond of the following formula (III) 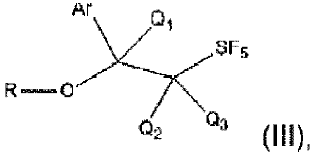 wherein the remainder Q₁ to Q₃ independently represent a deuterium atom, a cyano group, a trifluourmethyl group, a substituted or unsubstituted hydrocarbon group, which optionally contains one or more heteroatoms, or a substituted or unsubstituted aromatic residue, Ar is an aromatic residue, and R is a saturated or unsaturated, substituted or unsubstituted, unbranched or branched hydrocarbon group.

14. Method according to claim 13, wherein the vicinal pentafluorsulphane ether is a compound with a styrene basic structure.

15. Method according to any one of the preceding claims 12 to 14, wherein the strong Lewis acid in the reaction mixture represents BF₃·Et₂O.

## Revendications

1. Procédé de alkoxypentafluorosulfonylation photoredox d'un éduct à liaison carbone-carbone non saturée comprenant les étapes consistant à :
(A) fournir un mélange de réactions qui comprend l'éducte, un alcool ROH, un catalyseur photoredox ainsi qu'un hexafluorure de soufre, et
(B) irradier le mélange de réaction avec un rayonnement électromagnétique qui est la lumière UV et/ou la lumière d'une plage de longueurs d'onde du domaine visible,
R étant choisi parmi les groupes hydrocarburés, saturés ou non saturés, substitués ou non substitués, sans dérivation ou avec dérivation.

2. Procédé selon la revendication 1,
dans lequel
le catalyseur photoredox est un catalyseur photorédox avec une structure de base de N-Phénylphénothiazine.

3. Procédé selon la revendication 1 ou 2,
dans lequel
le catalyseur photoredox est une combinaison selon la formule (1) suivante : 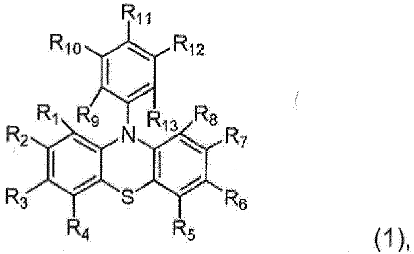 dans laquelle les radicaux R₁-R₁₃ sont indépendamment l'un de l'autre, un atome d'hydrogène, un atome de deutérium, un atome allogèné, un groupe cyano, un groupe nitro, un groupe trifluorométhyle, un atome de fluor, un groupe hydrocarburé, substitué ou non substitué qui contient, le cas échéant, un ou plusieurs hétéroatome ou un radical aromatique substitué ou non substitué.

4. Procédé selon l'une des revendications 1 à 3,
dans lequel
le catalyseur photorédox est N-phénylphénotiazine.

5. Procédé selon l'une des revendications 1 à 4,
dans lequel
la liaison carbone-carbone non saturée est une double liaison.

6. Procédé selon l'une des revendications 1 à 5,
dans lequel
l'éduct contient une combinaison de formule suivante : 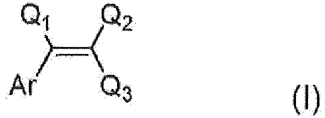 dans laquelle
les radicaux Q₁-Q₃ sont indépendamment l'un de l'autre, un atome d'hydrogène, un atome de deutérium, un atome cyano, un groupe trifluorométhyle, un groupe hydrocarburé substitué ou non substitué qui contient, le cas échéant, un ou plusieurs hétéroatomes ou un radical aromatique substitué ou non substitué et Ar est un radical aromatique.

7. Procédé selon l'une des revendications 1 à 6,
dans lequel
l'éduct est une combinaison avec une structure de base styrène.

8. Procédé selon l'une des revendications 1 à 7,
dans lequel
le rayonnement électromagnétique est une lumière d'une plage de longueurs d'onde visible ayant une longueur d'onde comprise entre 400 et 500 nm et/ou la lumière UV-A.

9. Procédé selon l'une des revendications 1 à 8,
dans lequel
le mélange de réaction contient en outre un acide de Lewis.

10. Procédé selon la revendication 9,
dans lequel
l'acide de Lewis ajouté au mélange de réaction est le tri-éthyl-borane.

11. Procédé selon l'une des revendications 1 à 10,
dans lequel
si le groupe R de l'alcool ROH est un groupe alcynylalkyle HCC (CH₂)ₙ-avec n>1, dans l'étape (B), à côté du pentafluor-sulfane-éther vicinal en forme 4-(Pentafluor-λ⁶-sulfanyl)-méthyl)-oxacycloalcane.

12. Procédé pour représenter de pentafluor-(éthylène)-λ⁶-sulfanes à partir des pentafluor (2-alkoxyéthyl)-λ⁶-sulfanes selon les revendications 1-10 comprenant les étapes consistant à :
(C) fournir un mélange de réaction qui comprend comme éduct un pentafluor-sulfényl-éther vicinal, un acide fort de Lewis et un solvant,
(D) agiter le mélange de réaction, et
(E) tremper le mélange de réaction avec une solution aqueuse et extraire le produit avec un solvant organique.

13. Procédé selon la revendication 12,
dans lequel
le pentafluor-sulfanyl-éther vicinal est une combinaison selon la formule (III) suivante : 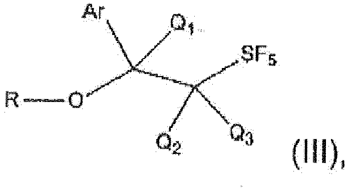 dans lequel
les radicaux Q₁-Q₃ sont indépendamment l'un de l'autre, un atome d'hydrogène, un atome de deutérium, un groupe cyano, un groupe trifluorométhyle, un groupe hydrocarburé substitué ou non-substitué qui contient, le cas échéant un ou plusieurs hétéroatome ou un résidu aromatique substitué ou non-substitué, Ar étant un radical aromatique et R est un groupe hydrocarburé, saturé ou non-saturé, substitué, ou non-substitué, dérivé ou non-dérivé.

14. Procédé selon la revendication 13,
dans lequel
le pentafluor-sulfanyl-éther est une combinaison avec une structure de base styrène.

15. Procédé selon l'une des revendications 12 à 14,
dans lequel
l'acide de Lewis dans le mélange de réaction est BF₃·Et₂O.
